(19) Europäisches Patentamt
European Patent Office
Office européen des brevets

(11) Publication number: **0 066 884**
**B1**

(12) # EUROPEAN PATENT SPECIFICATION

(45) Date of publication of patent specification: **14.05.86**

(51) Int. Cl.⁴: **A 61 K 9/50,** A 61 K 31/40

(21) Application number: **82105026.7**

(22) Date of filing: **08.06.82**

(54) **Oxygen-adsorbing and desorbing agent.**

(30) Priority: **10.06.81 JP 89312/81**

(43) Date of publication of application:
**15.12.82 Bulletin 82/50**

(45) Publication of the grant of the patent:
**14.05.86 Bulletin 86/20**

(84) Designated Contracting States:
**CH DE FR GB IT LI NL**

(56) References cited:

**CHEMICAL ABSTRACTS, vol. 88, no. 21, May 22, 1978, ref. no. 147805s, page 201, Columbus Ohio (US) M.A. KULISH et al.: "Study of the interaction of porphyrins and their metallocomplexes with phospholipid vesicles"**

**JOURNAL OF THE AMERICAN CHEMICAL SOCIETY, vol. 97, no. 6, March 19, 1975, J.P. COLLMAN et al.: "Picket fence porphyrins' synthetic models for oxygen binding hemoproteins", pages 1427-1437**

**CHEMICAL ABSTRACTS, vol. 91, no. 5, July 30, 1979, ref. no. 34427p, page 200, Columbus Ohio (US), B.E. HORSEY et al.: "Photochemistry of free base and metalloporphyrin complexes in monolayers"**

(73) Proprietor: **TAIHO PHARMACEUTICAL CO., LTD.**
**2-9, Kandatsukasa-machi Chiyoda-ku**
**Tokyo 101 (JP)**

(72) Inventor: **Hasegawa, Etsuo**
**13-69, Matsuoka, Kawauchi-cho**
**Tokushima-shi Tokushima-ken (JP)**
Inventor: **Matsushita, Yoh-ichi**
**7-3, Suketohonmachi, Tokushima-shi**
**Tokushima-ken (JP)**
Inventor: **Tsuchida, Eishun**
**1-141, Seki-machi, Nerima-ku**
**Tokyo (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

(56) References cited:

**CHEMICAL ABSTRACTS, vol. 96, no. 23, June 7, 1982, ref. no. 195504e, page 293, Columbus Ohio (US), HASEGAWA, ETSUO et al.: "Enzymic reduction of synthetic hemin to heme and its application to the study of oxygenation in aqueous medium"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 066 884 B1

**Description**

The invention relates to an oxygen-absorbing and desorbing agent consisting of a liposome.

The iron(II)-porphyrin complex present in hemoglobin or myoglobin adsorbs and desorbs oxygen molecules reversibly. In the prior art, many attempts have been made to synthesize a complex having oxygen-adsorbing and desorbing capacity analogous to that of the natural iron(II)-porphyrin complex. Typical examples of such attempts are found in articles such as J. P. Collman, Accounts of Chemical Research, *10*, 265 (1977); F. Basolo, B. M. Hoffman and J. A. Ibers, ibid., *8*, 384 (1975); and the like. Among others, the iron(II)-5,10,15,20-tetra(α,α,α,α-o-pivalamidophyenyl)porphyrin complex (hereinafter referred to as Fe(II)TpivPP) is reported to be capable of forming a stable oxygenated complex at room temperature [refer to J. P. Collman et al., Journal of the American Chemical Society, *97*, 1427 (1975)]. This iron(II)-porphyrin complex actually forms a stable oxygenated complex in aprotic solvents. However, if even a small amount of water is present, the iron(II)-porphyrin complex is oxidized immediately upon contact with oxygen. This converts the central iron atom from the bivalent to the trivalent state, so that the formation of an oxygenated complex becomes impossible.

Chemical Abstracts, Vol. 88, No. 21, May 22, 1978, reference number 147805 s and Vol. 51, No. 5, July 30, 1979, reference number 34427 p, describe an iron-porphyrin complex which is mixed with lectin. These references, however, do not disclose an imidazole ligand at the porphyrin complex. The presence of such an imidazole ligand is, however, very important for a reversibly oxygen-adsorbing and desorbing agent.

J. Amer. Chem. Soc., Vol. 97, No. 6, page 1427-1439, describes a conventional synthetic iron-porphyrin complex which is, however, oxidized in the presence of even small amounts of water, whereby it loses the function of reversibly binding oxygen molecules. In the above publication all oxygen adsorption-desorption tests described are conducted in water-free organic solvents, i.e. tetrahydrofurane, benzene, and dimethylformamide.

For this reason, there is still an eager search for an iron(II)-porphyrin complex which can yield an oxygenated complex at room temperature even in the presence of water.

Accordingly, it is an object of the present invention to provide an iron(II)-porphyrin complex system which can form a stable oxygenated complex at room temperature in an aqueous phase and, moreover, can adsorb or desorb oxygen reversibly in response to partial oxygen pressure.

According to the present invention the above object is accomplished by an oxygen-adsorbing and desorbing agent consisting of a liposome formed from at least one lipid or mixture of said lipid and cholesterol, said liposome having enclosed in a hydrophobic region thereof an iron(II)-5,10,15,20-tetra(α,α,α,α-o-substituted amidophenyl)porphyrin-imidazole complex having the formula

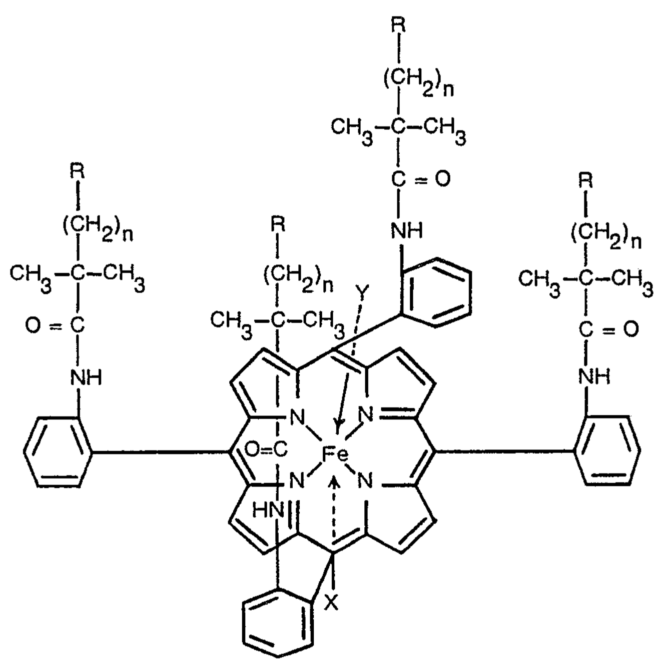

(I)

wherein R is a hydrogen atom, an alkoxycarbonyl group having 1 to 5 carbon atoms in the alkoxy moiety, a benzyloxycarbonyl group or a carboxyl group, n is an integer of 0 to 20, wherein when R is hydrogen, n is not 0, and each of X and Y is a substituted imidazole having the formula:

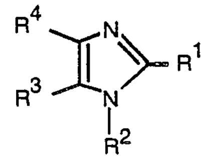

wherein $R^1$ is a hydrogen atom or alkyl group having one to three carbon atoms, and each of $R^2$, $R^3$ and $R^4$ is independently selected from a hydrogen atom or a hydrophobic group, at least one of $R^2$, $R^3$ and $R^4$ is a hydrophobic group, and wherein when $R^1$ is an alkyl group, Y is not present, said liposome being dispersed in an aqueous medium.

This invention can be more fully understood from the following detailed description when taken in conjunction with the accompanying drawings, in which:

Fig. 1 is a diagram illustrating two visible absorption spectra of the liposome-enclosed iron(II)-5,10,15,20-tetra[α,α,α,α-o-pivalamidophenyl]porphyrinmono(1-n-lauryl-2-methylimidazole) complex prepared in Example 1 according to the present invention wherein spectra *a* and *b* were recorded under atmospheres of nitrogen gas and oxygen gas, respectively; and

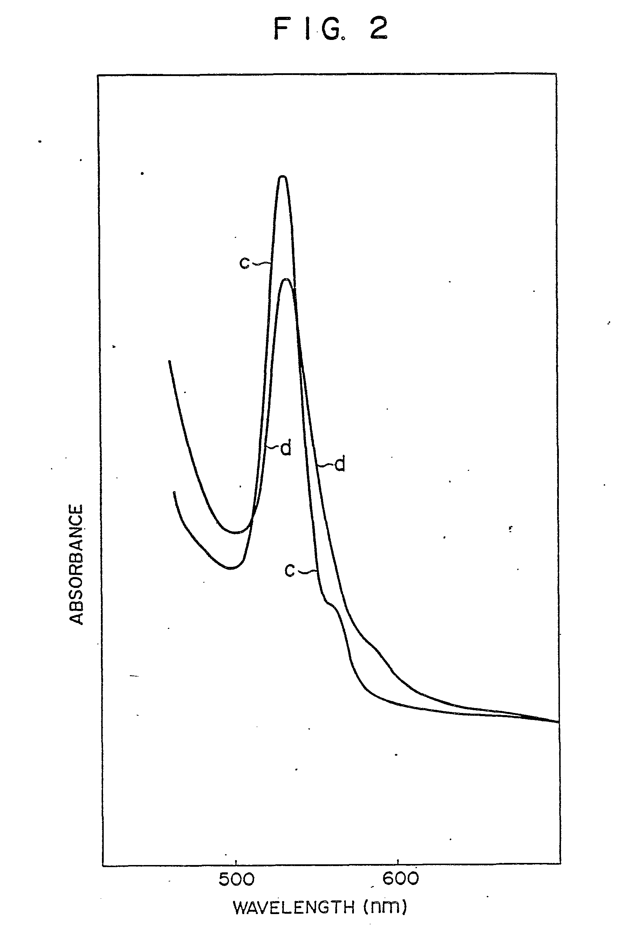

Fig. 2 is a diagram illustrating two visible absorption spectra of the liposome-enclosed iron(II)-5,10,15,20-tetra[α,α,α,α-o-(2',2'-dimethyl-4'-methoxycarbonylbutanamido)phenyl]porphyrin-bis(1-laurylimidazole) complex prepared in Example 16 according to the present invention wherein spectra *c* and *d* were recorded under atmospheres of nitrogen gas and oxygen gas, respectively.

Generally a porphyrin complex is highly hydrophobic and cannot be easily dissolved in water. Indeed an iron complex having porphyrin as a ligand may be uniformly dissolved or dispersed in water if there is added to water a synthetic surfactant such as a cationic surfactant including cetyltrimethyl ammonium chloride and dodecyl pyridinium chloride, and anionic surfactant including dodecyl sodium sulfate or a nonionic surfactant including poly oxyethylene nonylphenyl ether and fatty acid oxyethylene ester. However, the iron complex cannot carry oxygen. When such an oxygen carrier system is put to medical use, the poisonousness of the commercially available synthetic surfactant becomes problematical.

The inventors prepared a liposome, using a lipid, a mixture of lipids, or a mixture of one or more lipids and cholesterol. They further enclosed an iron(II) porphyrin-imidazole complex in the liposome thus prepared. Moreover, they successfully enclosed the complex in the hydrophobic region of liposome. The liposome having the complex enclosed in it was found to carry a lot of oxygen. This is the basic idea of the present invention.

The lipids which may be used in the present invention are: a phospholipid such as lecithin or phospatidyl choline, cephalin, phosphatidic acid, phosphatidyl serine and phosphatidyl ethanolamine, and sphingolipid such as sphingomyelin. These lipids are used, as mentioned, above, singly, in the form of a mixture thereof, or in the form of a mixture with cholesterol. A mixture of one or more lipids with cholesterol is preferred. The weight ratio of cholesterol to lipid is usually 1:2—20, preferably 1:3—10. Phosphatidyl choline, for example, may be not only a natural one such as egg yolk phosphatidyl choline or soyabean phosphatidyl choline but also a synthetic one such as dipalmitoyl phosphatidyl choline, distearoyl phosphatidyl choline or dimyristoyl phosphatidyl choline.

The molar ratio of a lipid, a mixture of lipids or a lipid-cholesterol mixture to iron porphyrin in the liposome solution ranges from 10 to 1,000, more preferably from 100 to 300.

An iron porphyrin complex itself can hardly adsorb oxygen through coordination and can hardly desorb oxygen. To make the iron porphyrin complex able to adsorb and desorb a lot of oxygen a basic ligand must be coordinated on the axis of the central iron ion. In the present invention, the axial ligand used for this purpose is the substituted imidazole having the general formula:

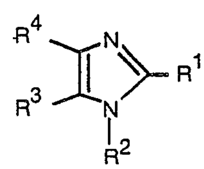

where $R^1$, $R^2$, $R^3$ and $R^4$ are as defined before.

Generally, $R^2$ or $R^3$ is the hydrophobic group. In the present invention, $R^2$ is the hydrophobic group in most cases. Such a hydrophobic group is a trityl group. Alternatively, it is a group which has the following general formula:

$$-(CH_2)_l-Z, \qquad \text{(IV)}$$

where l is 10 to 20 and Z is hydrogen or alkoxycarbonyl group having 1 to 5 carbon atoms in the alkoxy group. Hereinafter the term "hydrophobic group" will be used to designate any one of the above-mentioned substitutent groups.

The molar ratio of imidazole to iron-porphyrin in the liposome dispersion solution is 2 to 30, preferably

5 to 10, when $R^1$ is hydrogen. Said molar ratio is 10 to 100, preferably 30 to 100, when $R^1$ is a group other than hydrogen.

It is required that the substituted imidazole used in the invention would have group $R^2$ at 2-position and should have a hydrophobic group. Upon conducting experiments the inventors found out that a substituted imidazole having no hydrophobic group, for example Fe(II) Tpiv PP complex having 1,2-dimethylimidazole as an axial ligand, would be oxidized without forming a stable oxgenated complex even if it was enclosed in a lipid liposome. They also found that the complex was better enclosed in the liposome when the group used was more hydrophobic, for instance when an alkyl group having more carbon atoms was used. The more hydrophobic the group was, the more stable was the resultant oxygenated complex.

The iron porphyrin of formula (I) may be synthesized by utilizing the method disclosed in the article by J. P. Collman et al, Journal of the Americal Chemical Society *97* 1427 (1975). More specifically, 5,10,15,20-tetra(α,α,α,α-o-aminophenyl) porphyrin of the following formula (V) (hereinafter referred to as "α,α,α,α-Tam PP $H_2$") is reacted with at least 4 molar equivalents of a 2,2-dimethylcarboxylic acid chloride of the following formula (VI).

Formula (V):

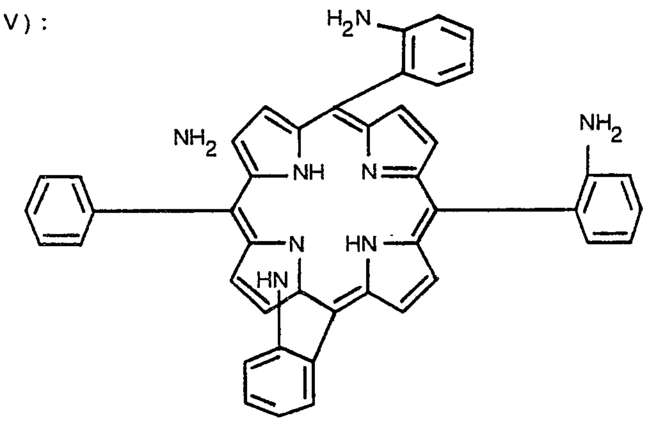

Formula (VI):

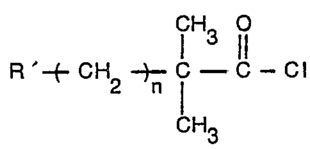

(where R′ is the R in formula (I) other than a carboxy group). Iron is then introduced into the thus obtained porphyrin by e.g. heating iron(II) bromide and the porphyrin in anhydrous tetrohydrofuran in the presence of a small amount of pyridine, thereby preparing the Fe(III)-porphyrin of formula (I) wherein R is other than a carboxy group. If R in formula (I) is a carboxy group, the corresponding iron porphyrin is synthesized by hydrolyzing the alkyl ester or benzyl ester groups in the iron-porphyrin obtained above wherein R is the alkoxycarbonyl or benzyloxy-carbonyl group.

The 2,2-dimethylcarboxylic acid chlorides of formula (VI) may be synthesized by reacting the corresponding carboxylic acid derivatives of the following formula (VII) with thionyl chloride, oxalic chloride, or triphenylphosphine/carbon tetrachloride:

Formula (VII):

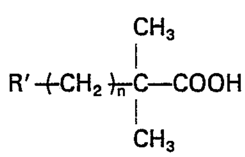

Carboxylic acid derivatives of formula (VII) may be produced by a method, a modification of the method developed by P. E. Pfeffer et al (see Journal of Organic Chemistry, *35,* 262 (1970)) if R′ is hydrogen. If R′ is alkoxylcarbonyl group or benzyloxycarbonyl group, the derivatives may be produced by a modification of the method developed by Y.N. Kuo et al (see Journal of the American Chemical Society, *93,* 6321 (1971)) or may be produced by reacting ω-bromoalkylcarboxylic acid alkyl ester with isobutyric acid dianion prepared by reacting lithium diisopropylamide and isobutyric acid.

The imidazole ligand having a hydrophobic group may be synthesized by thermally reacting 2-methylimidazole and an alkylbromide, neutralizing the product of the thermal reaction, preliminarily refining the product in a silica gel column and distilling the product under a reduced pressure. Trityl derivatives may be synthesized by adding trityl chloride to 2-methylimidazole silver salt, thermally reacting these substances, preliminarily distilling the product of the thermal reaction and recrystallizing the product.

The iron(II) porphyrin complex is enclosed in the liposome by the following method. First, in an inert

gas atmosphere of, for example, nitrogen gas or argon gas, Fe(III) TRPP.Br and an excessive amount of substituted imidazole (imidazole/porphyrin molar ratio = 2—100), for instance, are dissolved in a suitable solvent such as chloroform, dichloromethane, benzene or toluene. To the solution thus obtained an aqueous solution of a reducing agent (e.g. sodium dithionite) is added. The mixture of these solutions is shaken and stirred. As a result, the central iron of the iron porphyrin contained in the heterogeneous water-solvent system, which is trivalent, is reduced and becomes divalent. The organic phase is then collected and dehydrated. To the organic phase there is then added an excessive amount of a lipid, a mixture of lipids or a mixture of one or more lipids and cholesterol, so that in the resultant material the lipid/iron porphyrin molar ratio becomes 10 to 1,000, preferably 100 to 300. Thereafter, the solvent is removed by distillation under a reduced pressure, thereby forming a thin film of said material on the inner surface of a glass flask. The distillation should better be carried out while CO gas is being blown into the solution. The CO can easily be removed during the subsequent thermal dehydration in vacuo. In an inert gas atmosphere water or an aqueous solution is added to the solidified material. (The aqueous solution may be phosphate buffer solution, physiological saline, Krebs-Ringers solution, or a solution consisting of any of these solutions, a hematopoietics such as dextran, hydroxylethyl starch or albumine and amino acid, saccharide, vitamine or the like). The mixture of the aqueous solution and the material is shaken, thereby forming a liposome dispersion containing the iron(II) porphyrin complex which is enclosed in a multilayer liposome. Ultrasonic waves are applied on the liposome dispersion, thus converting the multilayer liposome into multilayer liposome particles of a smaller diameter or into single compartment liposome particles.

The above-mentioned reducing process using an aqueous solution of sodium dithionite may be replaced by the reducing process which uses palladium on carbon/hydrogen gas. More specifically, iron(II)porphyrin complex and imidazole ligand are dissolved in anhydrous dichloromethane or benze/methanol solution. To the solution thus prepared a small amount of palladium on carbon is added. Thereafter, hydrogen gas is blown into the solution, thus conducting reduction at room temperature. The iron(II) porphyrin complex solution is then shaken and filtered, thus removing palladium on carbon.

Further, according to the invention, after a dispersion containing iron(II) porphyrin-imidazole complex enclosed in liposome is prepared, the central iron can be reduced to iron(II) by an enzymatic reducing system (for example, the emzymatic reducing system may comprise β-nicotinamidoadenindinucleotido phosphoric acid ($NADP^+$), D-glucose-6-phosphate, glucose-6-phosphate dehydrogenase, ferre doxine and ferredoxine-NADP reductase) is added in such an amount that one or more moles of D-glucose-6-phosphate is present to one mol of iron(II) porphyrin complexes. The enzymatic reduction can be conducted in an aqueous medium whose pH value ranges from 5 to 9, thereby providing the desired liposome dispersion enclosing iron(II) porphyrin complex.

The liposome of this invention which may be produced by any method that is mentioned above encloses iron(II) porphyrin complex. Enclosed in the liposome, the iron(II) porphyrin complex can adsorb and desorb a lot of oxygen and remains stable at room temperature and even in the presence of water. The lipids used in great quantities for forming liposome has a good biocompatibility. The liposome according to the invention may be administered to living bodies as an oxygen-adsorbing and desorbing agent.

The present invention is further illustrated by the following examples.

Examples 1

Under an atmosphere of nitrogen gas, 1 mg ($8.7 \times 10^{-4}$ mmole) of Fe(III)TpivPP.Br and 11 mg ($4.3 \times 10^{-2}$ mmole) of 1-n-lauryl-2-methylimidazole were dissolved in 5 ml of toluene. To this solution was added 5 ml of an aqueous solution containing an excess of sodium dithionite. After the resulting mixture was shaken, carbon monoxide gas was bubbled therethrough.

After the mixture was allowed to settle, the toluene layer was collected and dehydrated by the addition of a small amount of Molecular Sieves 3 Å. To this toluene solution, in a flask, was added a solution of 0.13 g of egg yolk phosphatidylcholine in 2 ml of toluene which solution had previously been saturated with carbon monoxide gas. After the toluene was distilled off under reduced pressure, the thin film of solid material deposited on the inner wall of the flask was further exposed to reduced pressure at 100°C for 0.5 hour to remove any residual carbon monoxide. Then, 10 ml of a phosphate buffer solution (pH 7.0) free from oxygen gas was added to the flask*, and sonicated (20 kHz, 100 W) for 10 minutes under an atmosphere of nitrogen gas. Thus, there was obtained an aqueous dispersion of multiplayer liposomes having the iron(II)-5,10,15,20-tetra[α,α,α,α-o-pivalamidophenyl]porphyrinmono(1-n-lauryl-2-methylimidazole) complex enclosed therein. The visible absorption spectrum of this dispersion as recorded under an atmosphere of nitrogen gas exhibited absorption maxima at 532 and 562 mn and corresponded to the deoxygenated form of the complex, as illustrated by curve *a* in Fig. 1. The shape and λmax values of the spectrum are in good agreement with those of the spectra of the same complex which were recorded in anhydrous benzene or other solvents and reported in the literature.

The 1-n-lauryl-2-methylimidazole used in this example was synthesized as follows: A mixture of 19 g (0.23 mole) of 2-methylimidazole and 25 g (0.10 mole) of lauryl bromide was heated at 200°C for 10 hours. After this mixture was cooled, 500 ml of a 10% aqueous solution of potassium carbonate was added thereto, followed by stirring and extraction with two 300-ml portions of ether. The combined organic layer

* shaken to form an emulsion

was washed twice with 200-ml portions of a saturated aqueous solution of sodium hydrogen carbonate and then with a saturated aqueous solution of sodium chloride. Thereafter, the organic layer was collected and dried over anhydrous sodium sulfate. Subsequent evaporation gave a brown oil, which was purified by column chromatography using 500 g of silica gel as the absorbent and 10:1 mixture of chloroform and methanol as the solvent. Thus, 20 g of product was obtained in a 79% yield. This product was distilled under reduced pressure, and a distillate boiling at 156—158°C (4 mmHg) was used.

Mass spectrum: $M^+ = 250$.

$^1H$—NMR spectrum ($CDCl_3$, TMS), δ(ppm): 6.88 (d, J = 1 Hz, proton at the 4-position of the imidazole ring), 6.76 (d, J = 1 Hz, proton at the 5-position of the imidazole ring), 3.89 (2H, t, J = 7 Hz, N—$\underline{CH_2}$—$CH_2$— at the 1-position), 2.36 (3H, s, $\underline{CH_3}$— at the 2-position of the imidazole ring).

Infrared absorption spectrum (NaCl plates): 3,400, 2,930, 2,860, 1,525, 1,500, 1,465, 1,425, 1,375, 1,280, 1,145, 995, 720, 680 $cm^{-1}$.

Example 2

Spectrum *b* in Fig. 1 was recorded immediately after oxygen gas was bubbled, at room temperature, through the aqueous dispersion of liposomes (under an atmosphere of nitrogen gas) obtained in Example 1. This spectrum exhibits a single peak at 546 nm instead of the two peaks given by the deoxygenated form and agrees with the spectrum of the oxygenated form of the same Fe(II)-TpivPP complex which was recorded in anhydrous toluene and reported in the literature. By bubbling oxygen gas through the above dispersion containing the oxygenated complex for a short period of time, a shift from spectrum *b* to spectrum *a* was observed to confirm the reversible adsorption and desorption of oxygen. When the change with time of the visible absorption spectrum of the above oxygenated complex was traced, its half-life was found to be more than about 3 hours at room temperature.

Example 3

Employing the procedure of Example 1, a mixture was prepared from 3.6 mg ($3.15 \times 10^{-6}$ mole) of Fe(III)TpivPP.Br, 43 mg (50 molar equivalents) of 1-lauryl-2-methylimidazole, and 0.56 g (250 molar equivalents)* and then emulsified in 20 ml of physiological saline (pH 6.8). This emulsion was placed in an ice-water bath and sonicated (20 kHz, 150 W) under an atmosphere of nitrogen gas for 1.5 hours to obtain a substantially clear dispersion of liposomes having the reduced form of the complex enclosed therein.

Using this dispersion at room temperature, the complex was tested for reversible adsorption and desorption of oxygen and for stability (or change with time) of the oxygenated form. In the same manner as in Example 2, a visible absorption spectrum corresponding to the oxygenated complex was recorded by bubbling oxygen gas through the dispersion. Subsequently, the original spectrum of the deoxygenated form was obtained by bubbling nitrogen gas through the same dispersion, so that the reversible adsorption and desorption of oxygen was confirmed. The half-life of the above oxygenated complex was found to be more than 3 hours.

Example 4

A 5-ml portion of the dispersion of liposomes in reduced form which had been prepared in Example 3 was diluted with 5 ml of rat serum deaerated four times by the freeze-thawing method in a vacuum of $1.33 \times 10^{-4}$ mbar ($1 \times 10^{-4}$ torr). Then, the visible spectrum of the dilution was recorded using a reference cell containing the same rat serum diluted 1:1 with physiological saline. As stated before, the spectrum shifted from a curve with a peak at 546 nm to a curve with peaks at 535 and 562 nm or vice versa, according as oxygen was adsorbed or desorbed repeatedly. The half-life of the oxygenated complex was found to be more than 2.5 hours. Thus, no appreciable difference was noted between serum and physiological saline. This clearly indicates that the liposomes having the Fe(II)-porphyrin complex enclosed therein can exhibit oxygen-combining capacity in serum without undergoing any adverse effect.

Example 5

In a flask, 1 mg ($8.7 \times 10^{-4}$ mmole) of Fe(III)TpivPP.Br, 11 mg ($4.4 \times 10^{-2}$ mmole) of 1-n-lauryl-2-methylimidazole, and 125 mg of phosphatidylcholine were dissolved in 5 ml of benzene. This solution was concentrated at room temperature with the aid of a vacuum pump, so that a thin film of solid material was deposited on the inner wall of the flask. To this flask was added 10 ml of a 0.05 M phosphate buffer solution, which was cooled with ice and sonicated (20 kHz, 100 W) under an atmosphere of nitrogen gas for 30 minutes to obtain a dispersion of liposomes.

To the above dispersion of liposomes were added 0.5 mg of $NADP^+$, 3.0 mg of glucose-6-phosphate, 0.01 mg of ferredoxin, 0.02 mg of ferredoxin-NADP reductase, and 0.05 mg of catalase. Thereafter, nitrogen gas was bubbled through the dispersion for an hour, and a solution of 0.01 mg of glucose-6-phosphate dehydrogenase in 0.1 ml of a phosphate buffer solution (pH 7.0) was poured thereinto. While the resulting mixture was kept at 25°C under an atmosphere of nitrogen gas, the reduction of iron(III) to iron(II) was traced by recording its visible absorption spectrum at regular time intervals. As time went on, the absorbances at 535 and 560 nm increased owing to the formation of the iron(II)-5,10,15,20-tetra[α,α,α,α-o-pivalamido-phenyl]porphyrinmono(1-n-lauryl-2-methylimidazole) complex. The reduction was completed in approximately 5 hours.

* of egg yolk phosphatidylcholine

Subsequently, the above mixture was brought into contact with oxygen gas in the same manner as in Example 2. Immediately after that, a visible absorption spectrum ($\lambda_{max}$ = 544 nm) corresponding to the oxygenated complex was recorded. The half-life of this oxygenated complex as determined at 25°C was equal to that of the oxygenated complex obtained in Example 1.

Example 6

The procedure of Example 1 was repeated except that a combination of 0.13 g of egg yolk phosphatidylcholine and 0.014 g of cholesterol was used in place of the egg yolk phosphatidylcholine (0.13 g) alone. Thus, there was obtained an aqueous dispersion of multilayer liposomes having the iron(II)-5,10,15,20-tetra[α,α,α,α-o-pivalamidophenyl]porphyrin-mono(1-n-lauryl-2-methylimidazole) complex enclosed therein. The visible absorption spectrum of this dispersion as recorded under an atmosphere of nitrogen gas exhibited absorption maxima at 532 and 560 nm. In the same manner as in Example 2, a visible absorption spectrum ($\lambda_{max}$ = 544 nm) corresponding to the oxygenated complex was recorded by bubbling oxygen gas through the above dispersion. Furthermore, the original spectrum of the deoxygenated form was obtained by bubbling nitrogen gas through the same dispersion, so that the reversible adsorption and desorption of oxygen was confirmed. The half-life of the above oxygenated complex was found to be more than 3 hours.

Example 7

The procedure of Example 1 was repeated except that 1-trityl-2-methylimidazole was used in place of the 1-n-lauryl-2-methylimidazole. Thus, there was obtained an aqueous dispersion of liposomes having the iron(II)-5,10,15,20-tetra[α,α,α,α-o-pivalamidophenyl]porphyrin-mono(1-trityl-2-methylimidazole) complex enclosed therein. Its visible absorption spectrum exhibited absorption maxima at 532 and 560 nm. The formation of the oxygenated complex ($\lambda_{max}$ = 543 nm) was confirmed in the same manner as in Example 2.

The 1-trityl-2-methylimidazole used in this example was synthesized as follows: To a stirred suspension of 4.3 g (0.023 mole) of 2-methylimidazole silver salt in 50 ml of toluene was added 7.0 g (0.025 mole) of trityl chloride. The resulting mixture was heated under reflux for 10 hours. After being cooled to room temperature, the mixture was filtered to remove any insoluble matter (AgBr). The filtrate was concentrated to obtain a yellowish-brown solid material. This solid material was purified by column chromatography using 200 g of silica gel as the absorbent and a 20:1 mixture of chloroform and methanol as the solvent, and then recrystallized from a mixture of benzene and n-heptane. Thus, 3.34 g of product was obtained in a 45% yield.

Mass spectrum: $M^{+\cdot}$ = 324.

$^1$H—NMR spectrum ($CDCl_3$, TMS), δ (ppm): 7.00—7.40 (15 H, m, phenyl protons), 6.86 (1 H, d, J = 1 Hz, at the 4-position of the imidazole ring), 6.76 (1 H, d, J = 1 Hz, proton at the 5-position of the imidazole ring), 1.67 (3 H, s, —$C\underline{H}_3$ at the 2-position of the imidazole ring).

Infrared absorption spectrum (KBr disc): 3,350, 3,070, 3,030, 1,600, 1,520, 1,490, 1,450, 1,395, 1,305, 1,240, 1,180, 1,140, 1,070, 1,035, 1,000, 990, 910, 880, 865, 760, 750, 700 $cm^{-1}$.

Example 8

The procedure of Example 1 was repeated except that 0.18 g of dipalmitoylphosphatidylcholine was used in place of the egg yolk phosphatidylcholine (0.13 g) and the sonication was carried out on a bath at 60°C. Thus, there was obtained an aqueous dispersion of multilayer liposomes having the iron(II)-5,10,15,20-tetra[α,α,α,α-o-pivalamidophenyl]prophyrin- mono(1-n-lauryl-2-methylimidazole) complex enclosed therein. The visible absorption spectrum of this dispersion as recorded under an atmosphere of nitrogen gas exhibited absorption maxima at 532 and 562 nm. The formation of the oxygenated complex was confirmed in the same manner as in Example 2. The half-life of the oxygenated comples was found to be more than 3 hours.

Example 9

The procedure of Example 1 was repeated except that 1-stearyl-2-methylimidazole was used in place of the 1-n-lauryl-2-methylimidazole. Thus, there was obtained an aqueous dispersion of multiplayer liposomes having the iron(II)-5,10,15,20-tetra[α,α,α,α-o-pivalamidophenyl]porphyrin- mono(1-stearyl-2-methylimidazole) complex enclosed therein. The visible absorption spectrum of this dispersion as recorded under an atmosphere of nitrogen gas exhibited absorption maxima at 532 and 562 nm. The formation of the oxygenated complex was confirmed in the same manner as in Example 2. The half-life of this oxygenated complex was found to be more than 4 hours.

The 1-stearyl-2-methylimidazole used in this example was synthesized according to the method used to prepare 1-n-lauryl-2-methylimidazole described in Example 1, using stearyl bromide.

Mass spectrum: $M^{+\cdot}$ = 334.

$^1$H—NMR spectrum ($CDCl_3$, TMS), (ppm): 6.89 (d, 1 H), 6.80 (d, 1 H), 3.80 (t, 2 H), 2.37 (s, 3 H), 1.26 (2, 30 H), 0.88 (t, 3 H), 1.7 (t, 2 H).

Infrared absorption spectrum (KBr disk): 3,420, 2,980, 2,940, 2,870, 1,510, 1,480, 1,435, 1,385, 1,300, 1,290, 1,140, 1,110 $cm^{-1}$.

Elemental analysis (wt %):
Found: C, 79.01; H, 12.72; N, 8.31.
Calcd.: C, 79.0; H, 12.6; N, 8.4 for $C_{22}H_{42}N_2$.

Example 10

The procedure of Example 1 was repeated except that methyl 11-[1-(2-methyl)imidazolyl]undecanoate ($4.3 \times 10^{-2}$ mmole) was used in place of the 1-n-lauryl-2-methylimidazole. Thus, there was obtained an aqueous dispersion of liposomes having the iron(II)-5,10,15,20-tetra[α,α,α,α-o-pivalamidophenyl]-porphyrin-mono[methyl 11-{1-(2-methyl)imidazolyl}undecanoate] complex enclosed therein. The visible absorption spectrum of this dispersion as recorded under an atmosphere of nitrogen gas exhibited absorption maxima at 533 and 561 nm.

The methyl 11-[1-(2-methyl)imidazolyl]undecanoate used in this example was synthesized as follows: To a solution of 25 g of 11-bromoundecanoic acid in 100 ml of benzene was added 13 ml of oxalyl chloride. This solution was stirred at room temperature for 4 hours. After the addition of 50 ml of methanol, the resulting mixture was allowed to stand overnight and then worked up by conventional procedure to obtain 23 g of 11-bromoundecanoic acid methyl ester in an 87.5% yield. On the other hand, 8.2 g of 2-methylimidazole was slowly added to a suspension of 2.4 g of sodium hydride in 200 ml of dimethylformamide. After completion of the addition, the suspension was heated at 90°C for an hour, to which the previously prepared 11-bromoundecanoic acid methyl ester was added dropwise. The resulting mixture was stirred at 90°C for 2 hours and then worked up by conventional procedure. The resulting product was purified by silica gel column chromatography using a 9:1 (v/v) mixture of $CHCl_3$ and $CH_3OH$ as the solvent. Thus, 9.5 g of methyl 11-[1-(2-methyl)imidazolyl]undecanoate was obtained in a 41% yield.

Infrared absorption spectrum (liquid film): 1740 $cm^{-1}$ (νC=O, ester).

$^1$H—NMR spectrum ($CDCl_3$, TMS), (ppm): 1.28 (12 H, s, $>NCH_2CH_2(\underline{CH_2})_6CH_2CH_2CO_2—$), 1.68 (m, broad, $>NCH_2\underline{CH_2}(CH_2)_6\underline{CH_2}CH_2CO_2—$), 2.31 (2 H, t, $—CH_2CO_2—$), 2.37 (3 H, s, $—CH_3$ on the imidazole ring), 3.36 (3 H, s, $—COOH_3$), 3.80 (2 H, t, $>N\underline{CH_2}—$), 6.80 (1 H, d, proton at the 5-position of the imidazole ring), 6.90 (1 H, d, proton at the 4-position of the imidazole ring).

Mass spectrum: $M^{+\cdot}$ = 280.

Example 11

Using the aqueous dispersion of liposomes prepared in Example 10, the formation of an oxygenated complex was confirmed in the same manner as in Example 2. The visible absorption spectrum of this oxygenated complex exhibited an absorption maximum at 545 nm. The half-life of the oxygenated complex was found to be more than 3 hours.

Example 12

Employing the procedure described in an article by Philip E. Pfeffer et al. [The Journal of Organic Chemistry, *35*, 262 (1970)], 2,2-Dimethyltetradecanoic acid

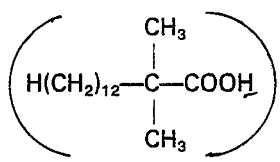

was synthesized using lauryl bromide. To 1.2 g of the resulting carboxylic acid was added 3 ml of thionyl chloride. This mixture was allowed to stand at room temperature for one day and then evaporated to dryness. Thus, 1.3 g of 2,2-dimethyltetradecanoyl chloride

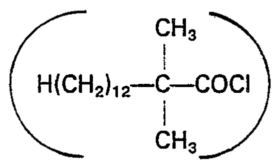

was obtained in the form of a colorless oil. Then, in the presence of tetrahydrofuran containing pyridine, the above acyl chloride was reacted with α,α,α,α-Tam PP·H2 in a molar ratio of 10:1. The resulting product was purified by silica gel column chromatography using a mixture of chloroform and petroleum ether as the solvent. Thus, 5,10,15,20-tetra[α,α,α,α-o-(2',2'-dimethyltetradecanamidophenyl)]porphyrin was obtained in a 20% yield.

Visible absorption spectrum ($CHCl_3$): $\lambda_{max}$ = 413, 507, 539, 582, 647 nm.

Infrared absorption spectrum (KBr disk): 2,960, 2,930, 2,850, 3,440, 1,690, 1,602, 1,580 $cm^{-1}$.

$^1$H—NMR spectrum ($CDCl_3$, δ (ppm): −2.60 (2 H, s, pyrrole >NH), −0.21 (24 H, s), 0.88 (12 H, t), 7.14—7.95 (16 H, m), 8.73 (4 H, s), 8.83 (8 H, s).

$^{13}$C—NMR spectrum ($CDCl_3$, TMS):

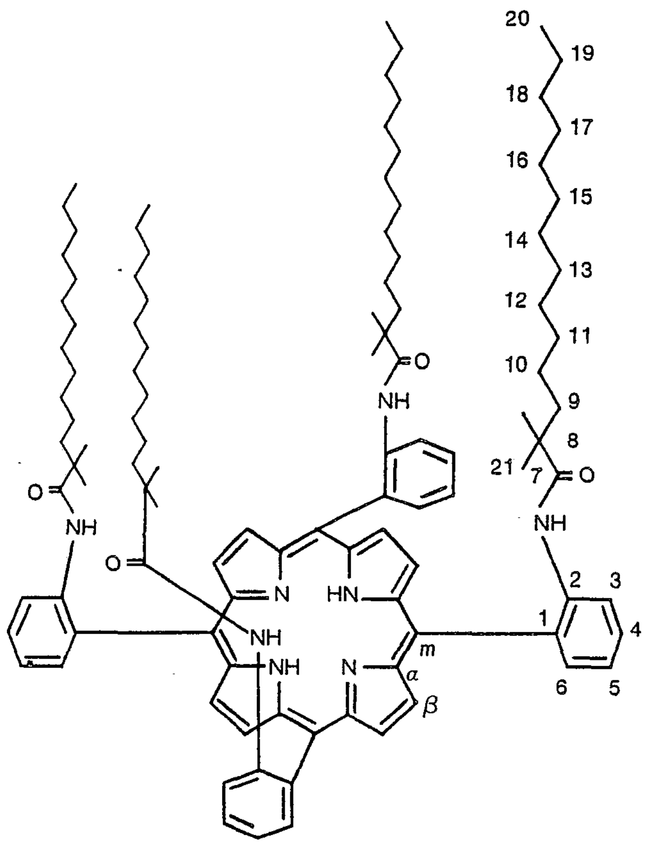

| Position | δ (ppm) | Position | δ (ppm) |
|---|---|---|---|
| α | 147.30 | 7 | 174.78 |
| β | 131.75 | 8 | 42.36 |
| m | 114.87 | 9 | 41.07 |
| 1 | 130.57 | 10—17 | 29.36, 29.65, 30.03 |
| 2 | 138.50 | 18 | 31.94 |
| 3 | 120.79 | 19 | 22.69 |
| 4 | 130.04 | 20 | 14.12 |
| 5 | 122.97 | 21 | 24.16 |
| 6 | 134.24 | | |

Elemental analysis (wt %):
Found: C, 79.21; H, 9.85; N, 7.02.
Calcd.: C, 79.64; H, 9.55; N, 6.88.

Field desorption mass spectrum: $(M+1)^{+\cdot}$ = 1627 (molecular weight for $C_{108}H_{154}N_8O_4$: 1626).

Under an atmosphere of nitrogen gas, the above porphyrin was reacted with anhydrous iron(II) bromide at elevated temperature in the presence of tetrahydrofuran containing a small amount of pyridine. Thus, the corresponding iron(III) porphyrin was synthesized.

Field desorption mass spectrum: $M^{+\cdot}$ = 1762 (molecular weight for $C_{108}H_{152}N_8O_4Fe_1Br_1$: 1762.4).

Elemental analysis (wt %): C, 73.60 (73.60); H, 8.46 (8.71); N, 6.21 (6.36). [The values given in parentheses represent the calculated values for $C_{108}H_{152}N_8O_4 \cdot Fe_1Br_1$.]

Infrared absorption spectrum (KBr disc): 3,440, 2,930, 2,860, 1,695, 1,585, 1,512, 1,440, 1,300, 1,000, 805, 755, 722 $cm^{-1}$.

## Example 13

A solution was prepared by dissolving 1.0 mg (5.68 × $10^{-4}$ mmole) of the bromo[5,10,15,20-tetra{α,α,α,α-o-(2′,2′-dimethyltetradecanamido)phenyl}porphyrinato]iron(III) synthesized in Example 12, 1.34 mg (5.68 × $10^{-3}$ mmole) of 1-laurylimidazole, and 20.8 mg (2.8 × $10^{-2}$ mmole) of dipalmitoylphosphatidylcholine in 10 ml of a 9:1 (v/v) mixture of benzene and methanol. A small amount of 10% Pd—C was added to this solution, through which hydrogen gas was bubbled for 20 minutes to reduce the iron atom. After the catalyst was removed by filtration, the solution was evaporated to dryness under reduced pressure. Under an atmosphere of nitrogen gas, a phosphate buffer solution (pH 7.2) which had been deaerated by the freeze-thawing method was added thereto and shaken to form an emulsion. Then, this emulsion was sonicated (20 kHz, 100 W) for 30 minutes to obtain a substantially clear dispersion of liposomes having the iron(II)-5,10,15,20-tetra[α,α,α,α-o-(2′,2′-dimethyltetradecanamido)phenyl]porphyrin-bis(1-laurylimidazole) complex enclosed therein. The visible absorption spectrum of this dispersion exhibited an absorption maximum at 532 nm and corresponded to the deoxygenated form of the complex. s soon as oxygen gas was bubbled through the dispersion, there occurred a shift to a spectrum ($\lambda_{max}$ = 536 nm) corresponding to the oxygenated complex. Furthermore, the original spectrum ($\lambda_{max}$ = 532 nm) was obtained by bubbling nitrogen gas through the same dispersion, so that the reversible adsorption and

desorption of oxygen was confirmed. The half-life of the above oxygenated complex was found to be more than 4 hours.

The 1-laurylimidazole used in the example was synthesized as follows: The procedure described in Example 1 for the synthesis of 1-n-lauryl-2-methylimidazole was repeated except that imidazole was used in place of the 2-methylimidazole. The resulting product was purified by distillation under reduced pressure, and a fraction boiling at 145—150°C (5 mmHg) was used.

$^1H$—NMR spectrum ($CDCl_3$, TMS): δ (ppm) = 0.88 (3 H, t, —$CH_3$), 1.26 (18 H, s, —$(CH_2)_9$—), 1.8 (2H, t, Im-C—$\underline{H_2}$—), 3.92 (2H, t, Im-$CH_2$—), 7.46 (1 H, s, proton at the 2-position of the imidazole ring), 6.90 (1 H, s, proton at the 5-position of the imidazole ring).

Infrared absorption spectrum (NaCl plate): 3,400, 3,120, 2,930, 2,860, 1,505, 1,465, 1,380, 1,285, 1,230, 1,110, 1,085, 1,030, 910, 810, 730, 670 $cm^{-1}$.

Example 14

2,2-Dimethyl-4-methoxycarbonylbutanoic acid was synthesized according to the procedure described in an article by Yu-Neng Kuo, Joseph A. Yahner and C. Ainsworth [Journal of the Americal Chemical Society, *93*, 6321—6323 (1971)]. Then, 1.40 g of the 2,2-dimethyl-4-methoxycarbonylbutanoic acid was reacted with 1.5 ml of thionyl chloride at room temperature for 12 hours. Thereafter, the reaction mixture was evaporated to dryness under reduced pressure to obtain 1.55 g of 2,2-dimethyl-4-methoxycarbonylbutanoyl chloride in the form of an oil. Its infrared spectrum (in chloroform) exhibited absorption bands at 2,990, 2,960, 1,790, 1,745, 1,480, 1,440, 1,300, 1,200, and 920 $cm^{-1}$, indicating the existence of an ester and an acyl chloride. A solution was prepared by dissolving 0.66 g (0.98 mmole) of α,α,α,α-Tam PP·$H_2$ in 50 ml of anhydrous tetrahydrofuran, and 1.54 g (8.00 mmole, 8.16 molar equivalents) of the above 2,2-dimethyl-4-methoxycarbonylbutanoyl chloride and 1.6 ml of pyridine were simultaneously added dropwise to the solution at 0°C. The resulting mixture was allowed to stand at room temperature for 4 hours. After the addition of 300 ml of chloroform, the mixture was washed twice with 200 ml portions of a 4% aqueous solution of sodium hydrogen carbonate. The chloroform layer was collected, dried over anhydrous sodium sulfate, and then evaporated to dryness under reduced pressure. The resulting crude product was purified by column chromatography using 150 g of silica gel as the adsorbent. After eluting with 1.5 liters of chloroform, a 50:1 (v/v) mixture of chloroform and methanol was used as solvent to elute the desired product. This product was recrystallized from a mixture of methylene chloride and methanol to obtain 0.79 g (0.61 mmole) of 5,10,15,20-tetra[α,α,α,α-o-(2′,2′-dimethyl-4′-methoxycarbonylbutanamido)phenyl]-porphyrin in a 62.5% yield.

Field desorption mass spectrum: $(M+1)^{\dot{+}}$ = 1299 (molecular weight for $C_{76}H_{82}N_8O_{12}$: 1298).

Infrared absorption spectrum (KBr disc): 3,440, 2,960, 1,732, 1,690, 1,582, 1,515, 1,450, 1,300, 1,200, 1,175, 970, 800, 760 $cm^{-1}$.

Visible absorption spectrum ($CHCl_3$): $\lambda_{max}$ = 418, 513, 543, 587, 644 nm.

$^1H$—NMR spectrum ($CDCl_3$, TMS): δ (ppm) = −2.66 (2 H, s), 0.22 (24 H, s), 0.62—1.20 (2 H, m), 1.42—2.00 (2 H, m), 2.94 (12 H, s), 7.15—8.97 (16 H, m), 8.79 (8 H, s).

$^1_3H$—NMR spectrum ($CDCl_3$):

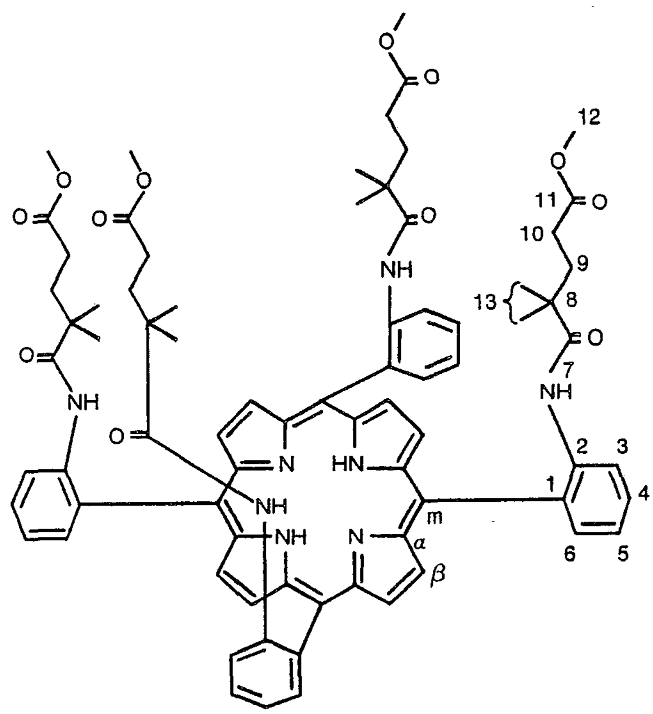

| Position | δ (ppm) | Position | δ (ppm) |
|---|---|---|---|
| α | 146.95 | 7 | 174.54 |
| β | 132.16 | 8 | 41.63 |
| m | 115.19 | 9 | 34.99 |
| 1 | 131.57 | 10 | 29.18 |
| 2 | 138.14 | 11 | 172.90 |
| 3 | 122.47 | 12 | 51.02 |
| 4 | 129.69 | 13 | 24.42 |
| 5 | 123.47 | | |
| 6 | 135.21 | | |

Elemental analysis (wt.%): C, 69.34 (69.45); H, 6.30 (6.51); N, 8.62 (8.42). [The values given in parentheses represent the calculated values for $C_{76}H_{82}N_8O_{12}\cdot CH_3OH$.]

Example 15

A solution was prepared by dissolving 0.454 g (0.349 mmole) of the 5,10,15,20-tetra[α,α,α,α-o-(2′,2′-dimethyl-4′-methoxycarbonylbutanamido)phenyl]porphyrin synthesized in Example 14 and 0.5 ml of pyridine in 60 ml of anhydrous tetrahydrofuran. This solution was heated under reflux for 30 minutes with nitrogen gas being bubbled therethrough. After the addition of 1.2 g (large excess) of ferrous bromide dihydrate ($Fe(II)Br_2\cdot 2H_2O$), the resulting mixture was heated at reflux for 4 hours under an atmosphere of nitrogen gas and then extracted with 300 ml of chloroform. The extract was washed twice with 200-ml portions of water. Thereafter, the chloroform layer was separated, dried over anhydrous sodium sulfate, and then evaporated to dryness under reduced pressure. The resulting black solid material was purified by column chromatography using 100 g of alumina as the absorbent and chloroform as the solvent. To the initially eluted fraction was added 2 ml of 48% hydrobromic acid. This mixture was shaken, dried over anhydrous sodium sulfate, and then evaporated to dryness under reduced pressure. The resulting product was recrystallized from a mixture of methanol and dilute hydrobromic acid, collected by filtration, and then dried in vacuo. Thus, 0.27 g (0.188 mmole) of bromo[5,10,15,20-tetra{α,α,α,α-o-(2′,2′-dimethyl-4′-methoxycarbonylbutanamido)phenyl}porphyrinato]iron(III) (hereinafter referred to as $Fe(TbutPP\cdot COOCH_3)Br$) was obtained in a 54% yield.

Silica gel thin-layer chromatography: Rf = 0.47 (solvent: chloroform/methanol = 20/1 (v/v)).

Field desorption mass spectrum: $(M+1)^{+\cdot}$ = 1433 (molecular weight for $C_{76}H_{80}N_8O_{12}FeBr$: 1432).

Infrared absorption spectrum (KBr disc): 3,420, 2,960, 1,730, 1,690, 1,580, 1,512, 1,440, 1,300, 1,200, 1,130, 1,000, 800, 760 $cm^{-1}$.

Visible absorption spectrum ($CHCl_3$): $\lambda_{max}$ = 416, 510, 584 nm.

Elemental analysis (wt.%): C, 63.26 (63.11); H, 5.73 (5.78); N, 7.73 (7.64). [The values given in parentheses represent the calculated values for $C_{76}H_{80}N_8O_{12}FeBr.CH_3OH$.]

A 30 mg portion of the $Fe(TbutPP.COOCH_3)Br$ was dissolved in 2 ml of methanol and to this solution was added 0.1 ml of 2N NaOH. The solution was stirred overnight at room temperature. Then, the solvent was removed by evaporation under reduced pressure and the residue was dissolved in 10 ml of water. The precipitates, which were obtained by adjusting the pH of the solution to 1.5, were collected and dried in vacuo at 60°C. Yield was 12 mg.

IR spectrum (KBr disc): 3,440, 2,970, 2,880, 1,710, 1,690, 1,585, 1,515, 1,445, 1,295, 1,205, 1,005 $cm^{-1}$.

Elemental analysis (wt%): C 65.19 (64.9), H 5.76 (5.4), N 8.26 (8.4) (The values in the parentheses were the calculated ones for $C_{72}H_{72}N_8O_{12}FeCl$.)

## Example 16

The procedure of Example 13 was repeated except that 0.82 g (5.68 × $10^{-4}$ mmole) of bromo[5,10,15,20-tetra{α,α,α,α-o-2′,2′-dimethyl-4′-methoxycarbonylbutanamido)phenyl}porphyrinato]-iron(III) was used in place of the bromo[5,10,15,20-tetra{α,α,α,α-o-(2′,2′-dimethyltetra-decanamido)phenyl}porphyrinato]iron(III). Thus, there was obtained an aqueous dispersion of liposomes having the iron(II)-5,10,15,20-tetra[α,α,α,α-o-(2′,2′-dimethyl-4′-methyoxycarbonylbutanamido)phenyl]-porphyrin-bis(1-laurylimidazole) complex enclosed therein. The visible absorption spectrum of this dispersion exhibited an absorption maximum at 532 nm and corresponded to the deoxygenated form of the complex. As soon as oxygen gas was bubbled through the dispersion, there occurred a shift to a spectrum ($\lambda_{max}$ = 536 nm) corresponding to the oxygenated complex. The half-life of this oxygenated complex was found to be 1 hour.

## Example 17

A solution of 14 ml (0.10 mole) of diisopropylamine in 150 ml of anhydrous tetrahydrofuran was cooled to −40°C under an atmosphere of nitrogen gas. Then, 43 g (0.10 mole) of a 15% (w/w) solution of n-butyl lithium in hexane was added dropwise thereto. The resulting mixture was allowed to stand for an hour to form litium diisopropylamide. Then, 4.73 ml (0.05 mole) of isobutyric acid was added dropwise to the mixture at a sufficiently low rate to maintain its temperature at −40°C or below, followed by the addition of 10 ml of hexamethylphosphoric triamide. Thereafter, the mixture was slowly warmed to 35°C, stirred at that temperature for 2 hours, and cooled again to −60°C. After 16 g (0.05 mole) of 11-bromoundecanoic acid t-butyl ester was added dropwise, the mixture was slowly warmed to room temperature and allowed to stand for one day. Then, the mixture was poured into 300 ml of ice water, which was adjusted to pH 2.0 with concentrated hydrochloric acid and then extracted twice with 200--ml portions of dichloromethane. The extract was washed twice with 100-ml portions of dilute hydrochloric acid and then twice with 70-ml portions of water. Thereafter, the organic layer was separated, dried over anhydrous sodium sulfate, filtered, and evaporated to dryness under reduced pressure. The resulting oily residue was purified by silica gel column chromatography using chloroform as the solvent. Thus, 3.7 g (0.11 mole) of 12-t-butoxycarbonyl-2,2-dimethyldodecanoic acid was obtained in an 11% yield.

Silica gel thin-layer chromatography: Rf = 0.50 (solvent: chloroform/ether = 4/1 (v/v)).

Mass spectrum: $(M+1)^{+}$ = 329 (molecular weight for $C_{19}H_{36}O_4$: 328).

Infrared absorption spectrum ($CCl_4$): 3,300—2,800 (broad), 2,990, 2,940, 2,860, 1,732, 1,705, 1,470, 1,370, 1,154 $cm^{-1}$.

$^1H$—NMR spectrum ($CDCl_3$, TMS): δ (ppm) = 1.18 (6H, s), 1.26 (14H, s), 1.44 (9H, s), 2.21 (2H, t, J = 7.1 Hz)

$^{13}C$—NMR spectrum ($CDCl_3$):

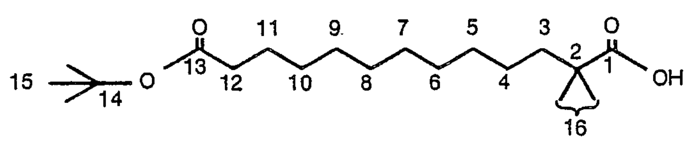

| Position | δ (ppm) | Position | δ (ppm) |
|---|---|---|---|
| 1 | 184.67 | 11 | 25.10 |
| 2 | 42.13 | 12 | 35.61 |
| 3 | 40.54 | 13 | 173.34 |
| 4 | 24.83 | 14 | 79.87 |
| 5 | 30.09 | 15 | 28.12 |
| 6—10 | 29.50, 29.41<br>29.27, 29.06 | 16 | 24.95 |

Elemental analysis (wt.%): C, 69.32 (69.47); H, 10.89 (11.05).

[The values given in parentheses represent the calculated values for $C_{19}H_{36}O_4$.]

Example 18

A solution was prepared by dissolving 2.20 g (6.7 mmole) of the 12′-butoxycarbonyl-2′,2′-dimethyl-dodecanoic acid synthesized in Example 7 and 2.3 g (8.8 mmole) of triphenylphosphine in 20 ml of anhydrous carbon tetrachloride, and then heated under reflux for 12 hours. After being cooled to 10°C, this solution was filtered to remove any insoluble matter (i.e., triphenylphosphine oxide). The filtrate was evaporated to dryness under reduced pressure to obtain an acid chloride in the form of an oil. This acid chloride and 0.8 ml of pyridine were simultaneously added dropwise to a solution of 0.20 g (0.30 mmole) of α,α,α,α-TamPP.$H_2$ in 50 ml of anhydrous tetrahydrofuran. This addition was carried out at room temperature over a period of 30 minutes. The resulting mixture was stirred for 4 hours, evaporated to dryness under reduced pressure, and then extracted with 200 ml of chloroform. The extract was washed twice with 100-ml portions of water, 100 ml of a saturated aqueous solution of sodium hydrogen carbonate, and then 50 ml of a saturated aqueous solution of sodium chloride. Thereafter, the chloroform layer was separated, dried over anhydrous sodium sulfate, filtered, and then evaporated to dryness under reduced pressure. The resulting residue was purified by silica gel column chromatography using chloroform as the solvent, and then alumina column chromatography using a 1:1 mixture of n-hexane and ether as the solvent. Finally, a reddish-brown solid material was isolated by preparative silica gel thin-layer chromatography using a 1:1 mixture of benzene and ether as the solvent. Thus, 0.042 g (0.022 mmole) of 5,10,15,20-tetra[α,α,α,α-o-(12′-t-butoxycarbonyl-2′,2′-dimethyldodecanamido)phenyl]porphyrin was obtained in a 7.3% yield.

Silica gel thin-layer chromatography: Rf = 0.39 (solvent: benzene/ether = 7/1).

Field desorption mass spectrum: $(M+1)^{+}$ = 1915 (molecular weight for $C_{120}H_{170}N_8O_{12}$: 1914).

Infrared absorption spectrum ($CCl_4$): 3,450, 3,330, 2,980, 2,940, 2,860, 1,735, 1,695, 1,590, 1,520, 1,450, 1,160, 973, 805, 762 $cm^{-1}$.

Visible absorption spectrum ($CHCl_3$): $\lambda_{max}$ = 417, 510, 543, 585, 624 nm.

$^1H$—NMR spectrum ($CDCl_3$, TMS): δ (ppm) = −2.60 (2H, s), −0.23 (24H, s), 1.26 (14H, brs), 1.45 (36H, s), 2.21 (8H, t, J = 7.1 Hz), 7.12 (4H, s), 7.50—8.72 (16H, m), 8.82 (8H, s).

$^{13}C$—NMR spectrum ($CDCl_3$):

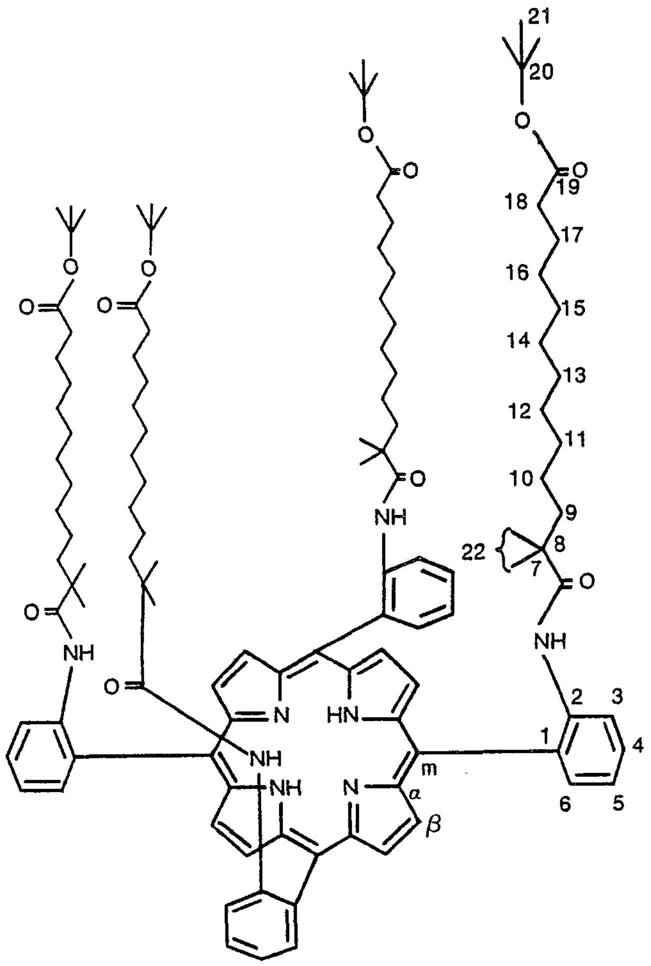

| Position | δ (ppm) | Position | δ (ppm) |
|---|---|---|---|
| α | 146.91 | 7 | 174.72 |
| β | 131.71 | 8 | 42.33 |
| m | 114.84 | 9 | 41.10 |
| 1 | 130.54 | 10 | 24.92 |
| 2 | 138.54 | | 30.00 |
| | | | 29.65 |
| 3 | 120.74 | 11–16 | 29.56 |
| | | | 29.44 |
| 4 | 130.04 | | 22.30 |
| | | | 29.12 |
| 5 | 122.97 | | |
| 6 | 134.21 | 17 | 25.10 |
| | | 18 | 35.61 |
| | | 19 | 173.81 |
| | | 20 | 79.81 |
| | | 21 | 28.12 |
| | | 22 | 24.13 |

Elemental analysis (wt.%): C, 74.98 (75.19); H, 8.96 (8.94); N, 5.98 (5.85). [The values given in parentheses represent the calculated values for $C_{12}H_{170}N_8O_{12}$.]

Example 19

A solution was prepared by dissolving 0.038 g (0.020 mmole) of 5,10,15,20-tetra[α,α,α,α-o-[12'-t-butoxycarbonyl-2',2'-dimethyldodecanamido)phenyl]porphyrin and 0.05 ml of pyridine in 20 ml of anhydrous tetrahydrofuran, and then deaerated by bubbling nitrogen gas therethrough for an hour. After the addition of 0.10 g (large excess) of ferrous bromide dihydrate ($FeBr_2.2H_2O$), this solution was heated under reflux for 3 hours. One hundred milliliters of ether was added to the solution, and the resulting mixture was washed twice with 100-ml portions of water. Thereafter, the organic layer was separated, dried over anhydrous sodium sulfate, and then evaporated to dryness under reduced pressure. The resulting residue was purified by alumina column chromatography using dichloromethane as the solvent, and then treated with 48% hydrobromic acid. Thus, 0.038 g (0.19 mmole) of bromo[5,10,15,20-tetra{α,α,α,α-o-(t-butoxycarbonyl-2',2'-dimethyldodecanamido)phenyl}porphyrinato]iron(III) (hereinafter referred to as Fe(TdodPP.COOtBu)Br) was obtained in a 92% yield.

Infrared absorption spectrum ($CCl_4$): 3,440, 2,940, 2,860, 1,730, 1,690, 1,580, 1,510, 1,440, 1,150, 1,000 $cm^{-1}$.

Visible absorption spectrum ($CHCl_3$): $\lambda_{max}$ = 415, 507, 580, 650 nm.

Elemental analysis (wt.%): C, 68.92 (69.15); H, 8.08 (8.14); N, 5.32 (5.35). [The values given in parentheses represent the calculated values for $C_{120}H_{168}N_8O_{12}FeBr.1/2CH_2Cl_2$.]

Example 20

The procedure of Example 13 was repeated except that 1.16 mg (5.68 × $10^{-4}$ mmole) of the bromo[5,10,15,20-tetra{α,α,α,α-o-(12′-t-butoxycarbonyl-2′,2′-dimethyldodecanamido)phenyl}-porphyrinato]iron(III) synthesized in Example 19 was used in place of the bromo[5,10,15,20-tetra{α,α,α,α-o′(2′,2′-dimethyltetradecanamido)-phenyl}porphyrinatol]iron(III). Thus, there was obtained an aqueous dispersion of liposomes having the iron(II)-5,10,15,20-tetra[α,α,α,α-o-(12′-t-butoxycarbonyl-2′,2′-dimethyl-dodecanamido)phenyl]porphyrin-bis(1-laurylimidazole) complex enclosed therein. The visible absorption spectrum of this dispersion exhibited an absorption maximum at 532 nm and corresponded to the deoxygenated form of the complex. As soon as oxygen gas was bubbled through the dispersion, there occurred a shift to a spectrum ($\lambda_{max}$ = 536 nm) corresponding to the oxygenated complex. The half-life of this oxygenated complex was found to be 1 hour.

Example 21

A solution was prepared in a vessel by dissolving 1 mg (4.88 × $10^{-4}$ mmole) of the Fe(TdodPP.COOtBu)Br synthesized in Example 19, 1.15 mg (4.9 × $10^{-3}$ mmole) of 1-laurylimidazole, and 73 mg (0.10 mmole) of egg yolk phosphatidylcholine in a 9:1 (v/v) mixture of chloroform and methanol. By evaporating this solution to dryness under reduced pressure, a thin film of solid material was deposited on the inner wall of the vessel and dried in vacuo for 4 hours. Then, 10 ml of physiological saline solution was added to the vessel and shaken to form an emulsion. Subsequently, this emulsion was sonicated (20 kHz, 100 W) for 40 minutes to obtain a substantially clear aqueous dispersion of liposomes. After this dispersion was deaerated by bubbling nitrogen gas therethrough for an hour, 1 ml of deaerated physilogical saline solution containing 0.5 mg of $NADP^+$, 3.0 mg of glucose-6-phosphate, 0.01 mg of ferredoxin, 0.02 mg of ferredoxin-NADP reductase, 0.05 mg of catalase, and 0.01 mg of glucose-6-phosphate dehydrogenase was added thereto. The resulting mixture was allowed to stand at room temperature for 5 hours under an atmosphere of nitrogen gas to reduce the iron atom. Thus, there was obtained an aqueous dispersion of liposomes having the iron(II)-5,10,15,20-tetra[α,α,α,α-o-(12′-t-butoxycarbonyl-2′,2′-dimethyl-dodecanamido)-phenyl]porphyrin-bis(1-laurylimidazole) complex enclosed therein. The visible absorption spectrum of the deoxygenated form of this complex exhibited absorption maxima at 426 and 532 nm, while that of the oxygenated complex obtained by bubbling oxygen gas through the dispersion had $\lambda_{max}$ values of 423 and 536 nm. Moreover, the reversible adsorption and desorption of oxygen was confirmed. The half-life of the oxygenated complex was found to be 2 hours.

Example 22

A 0.020-g portion of the Fe(TdodPP.COOtBu)Br synthesized in Example 19 was dissolved in 3 ml of trifluoroacetic acid. This solution was cooled in an ice-water bath, stirred for 6 hours to hydrolyze the Fe(TdodPP.COOtBu)Br, and then evaporated to dryness under reduced pressure. The resulting residue was dried in vacuo to obtain 0.0175 g of bromo[5,10,15,20-tetra{α,α,α,α-o-(12′-carboxy-2′,2′-dimethyldodecan-amido)phenyl}porphyrinato]iron(III) (hereinafter referred to as Fe(TdodPP.COOH)Br) in a 100% yield.

Infrared absorption spectrum ($CHCl_3$): 3,440, 2,930, 2,860, 1,710, 1,690, 1,582, 1,515, 1,440, 1,295, 1,160, 1,000 $cm^{-1}$. [The absorption band at 1,710 $cm^{-1}$ disappears on addition of triethylamine.]

Visible absorption sectrum ($CHCl_3$): $\lambda_{max}$ = 415, 507, 580, 652 nm.

Elemental analysis (wt.%): C, 68.12 (68.40); H, 7.42 (7.51); N, 6.24 (6.14). [The values given in parenthese represent the calculated values for $C_{104}H_{136}N_8O_{12}FeBr$.]

Fe(TdodPP.COOH)Br can be made soluble in aqueous media (such as water, a 0.1 M phosphate buffer solution (pH 7.2), physiological saline, and the like) by converting this carboxylic acid to its tetrasodium salt.

Example 23

The procedure of Example 21 was repeated except that the Fe(TdodPP.COOH)Br synthesized in Example 22 was used as an iron(III) porphyrin. Thus, there was obtained an aqueous dispersion of liposomes having the iron(III)-5,10,15,20-tetra[α,α,α,α-o-(12′-carboxyl-2′,2′-dimethyldodecanamido)-phenyl]porphyrin-bis(1-laurylimidazole) complex enclosed therein. The visible absorption spectrum of the deoxygenated form of this complex exhibited absorption maxima at 426 and 532 nm, while that of the oxygenated complex had $\lambda_{max}$ values of 423 and 536 nm. The half-life of the oxygenated comples was found to be 1 hour.

Example 24

2,2-Dimethylmalonic acid monomethyl ester monochloride (b.p. 58—61°C/13 mmHg) was synthesized according to the procedure described in an article by J. Bűchi, G. Enézian, H. Eichenberger and R. Lieberherr [Helvetica Chemica Acta, *35,* 75—82 (1952)]. To a solution of 0.50 g of α,α,α,α-TamPP.$H_2$ and 2 ml of pyridine in 50 ml of anhydrous tetrahydrofuran was added 1.5 g (9.12 molar equivalents) of the 2,2-dimethylmalonic acid monomethyl ester monochloride over a period of 10 minutes. The resulting mixture was allowed to stand at room temperature for 4 hours, added with 100 ml of 14% aqueous solution of sodium hydrogen carbonate, and then extracted with 200 ml of chloroform. The extract was washed twice with 200-ml portions f a 4% aqueous solution of sodium hydrogen carbonate. Thereafter, the chloroform layer was collected, dried over anhydrous sodium sulfate, filtered, and then evaporated to dryness under reduced pressure. The resulting oily residue was purified by column chromatography using 50 g of silica gel as the adsorbent and chloroform as the solvent. The initially eluted fraction was collected and concentrated under reduced pressure. The resulting purple needles were recrystallized from a mixture of chloroform and ether, collected by filtration, washed with ether, and then dried in vacuo. Thus, 0.74 g of 5,10,15,20-tetra[α,α,α,α-o-(2',2'-dimethyl-2'-methoxycarbonylacetamido)-phenyl]porphyrin was obtained in an 84% yield.

Field desorption mass spectrum: $(M+H)^{+}$ = 1187 (molecular weight for $C_{68}H_{66}N_8O_{12}$: 1186).

Infrared absorption spectrum (KBr disc): 3,380, 1,750, 1,695, 1,590, 1,525, 1,450, 1,160, 1,140, 970, 810, 765 $cm^{-1}$.

Visible absorption spectrum ($CHCl_3$): $\lambda_{max}$ = 418, 512, 544, 586, 645 nm.

$^1H$—NMR spectrum ($CDCl_3$): δ (ppm) = −2.54 (2H, s), 0.61 (24H, s), 2.04 (12H, s), 7.4—8.7 (16H, m), 8.80 (8H, s).

$^{13}C$-NMR spectrum ($CDCl_3$):

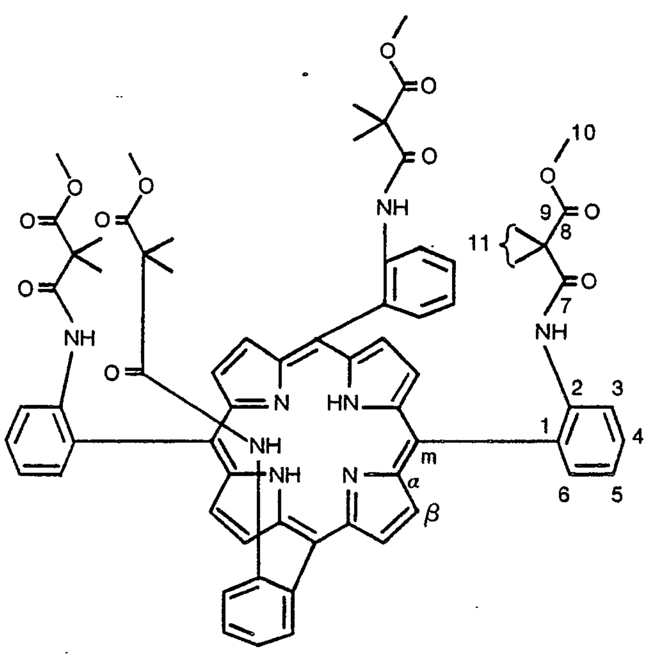

| Position | δ (ppm) | Position | δ (ppm) |
|---|---|---|---|
| α | 147.0 | 7 | 173.08 |
| β | 131.39 | 8 | 50.14 |
| m | 114.89 | 9 | 169.32 |
| 1 | 131.39 | 10 | 51.43 |
| 2 | 138.09 | 11 | 22.72 |
| 3 | 121.53 | | |
| 4 | 129.81 | | |
| 5 | 123.35 | | |
| 6 | 134.92 | | |

Elemental analysis (wt.%): C, 68.74 (68.79): H, 5.75 (5.60); N, 9.17 (9.44). [The values given in parentheses represent the calculated values for $C_{68}H_{66}N_8O_{12}$.]

Iron was introduced as follows: A solution was prepared by dissolving 0.38 g of 5,10,15,20-tetra[α,α,α,α-o-(2',2'-dimethyl-2'-methoxycarbonylacetamido)phenyl]porphyrin and 0.5 ml of pyridine in 40 ml of anhydrous tetrahydrofuran, and then deaerated by bubbling nitrogen gas therethrough for 2 hours. After the addition of 1.2 g (large excess) of ferrous bromide dihydrate ($FeBr_2.2H_2O$), this solution was heated under reflux for 4 hours and then extracted with 200 ml of chloroform. The extract was washed twice with 100-ml portions of water. Thereafter, the chloroform layer was separated, dried over anhydrous sodium sulfate, and then evaporated to dryness under reduced pressure. The resulting black solid material was purified by alumina column chromatography using chloroform as the solvent. The initially eluted fraction was collected, shaken with 3 ml of 48% hydrobromic acid, dried over anhydrous sodium sulfate,

filtered, and then evaporated to dryness under reduced pressure. The resulting product was recrystallized from a mixture of chloroform, ether, and petroleum ether, collected by filtration, and then dried in vacuo. Thus, 0.22 g of bromo[5,10,15,20-tetra{α,α,α,α-o-(2',2'-dimethyl-2'-methoxycarbonylacetamido)phenyl}-porphyrinato]iron(III) (hereinafter referred to as Fe(TacePP.$COOCH_3$)Br) was obtained in a 52% yield.

Field desorption mass spectrum: $(M+H)^{+\cdot}$ = 1321 (molecular weight $C_{68}H_{64}N_8O_{12}FeBr$: 1320).

Infrared absorption spectrum (KBr disc): 3,380, 1,750, 1,695, 1,590, 1,525, 1,450, 1,160, 1,140, 970, 810, 756 $cm^{-1}$.

Visible absorption spectrum ($CHCl_3$): $\lambda_{max}$ = 418, 512, 544, 586, 645 nm.

Elemental analysis (wt.%): C, 60.84 (60.86); H, 4.83 (4.79); N, 8.27 (8.30). [The values given in parentheses represent the calculated values for $C_{68}H_{64}N_8O_{12}FeBr.1/3CHCl_3$.]

Example 25

The procedure of Example 13 was repeated except that the Fe(TacePP.$COOCH_3$)Br synthesized in Example 24 was used as an iron(III) porphyrin. Thus, there was obtained an aqueous dispersion of liposomes having the iron(II)-5,10,15,20-tetra[α,α,α,α-o-(2',2'-dimethyl-2'-methoxycarbonylacetamido)-phenyl]porphyrin-bis(1-laurylimidazole) complex enclosed therein. The visible absorption spectrum of this dispersion exhibited absorption maxima at 429 and 534 nm. As soon as oxygen gas was bubbled through the dispersion, new absorption peaks appeared at 425 and 538 nm. These absorption peaks shifted reversibly to 429 and 534 nm when nitrogen gas was bubbled through the dispersion. The formation of the oxygenated complex was confirmed in this manner. The half-life of this oxygenated complex was found to be 1 hour.

Example 26

3-Benzyloxycarbonyl-2,2-dimethylpropionic acid was synthesized as follows: A solution of 20 g of 2,2-dimethylsuccinic anhydride in 100 ml of benzyl alcohol was cooled in an ice-water bath. After the addition of a few droplets of phenolphthalein, this solution was neutralized with a ca. 20% solution of sodium benzylate in benzyl alcohol until a red color appeared. After the benzyl alcohol was distilled off under reduced pressure, the resulting oily residue was dissolved in water, adjusted to pH 4.0 with a saturated aqueous solution of sodium dihydrogen phosphate, and then extracted twice with 200-ml portions of chloroform. The extract was dried over anhydrous sodium sulfate and then evaporated to dryness under reduced pressure. The resulting residue was recrystallized twice from a mixture of benzene and n-hexane, and then dried in vacuo. Thus, 23 g of 3-benzyloxycarbonyl-2,2-dimethylpropionic acid was obtained in a 62% yield.

Mass spectrum: $M^{+\cdot}$ = 236 (molecular weight for $C_{13}H_{16}O_4$: 236).

Infrared absorption spectrum (KBr disc): 2,980, 1,730, 1,707, 1,480, 1,180, 1,125 $cm^{-1}$.

$^1H$—NMR spectrum ($CDCl_3$): δ (ppm) = 1.30 (6H, s), 2.66 (2H, s), 5.11 (2H, s), 7.35 (5H, s).

A solution was prepared by dissolving 6.0 g of the above 3-benzyloxycarbonyl-2,2-dimethylpropionic acid and 7.99 g (1.2 molar equivalents) of triphenylphosphine in anhydrous carbon tetrachloride. This solution was heated under reflux for 20 hours and then allowed to cool. After the precipitated triphenylphosphine oxide was removed by filtration, the solution was concentrated to obtain 3-benzyloxycarbonyl-2,2-dimethylpropionyl chloride in the form of an oil. Without further purification, this acyl chloride was reacted with 0.50 g of α,α,α,α-TamPP.$H_2$ and 2 ml of pyridine in anhydrous tetrahydrofuran. This reaction was carried out under the same conditions as in Example 24, and the reaction mixture was worked up in the same manner as in Example 24. The resulting product was purified by silica gel column chromatography using a 50:1 mixture of chloroform and methanol as the solvent, and then recrystallized from a mixture of dichloromethane and methanol. Thus, 0.68 g of 5,10,15,20-tetra[α,α,α-(o-3'-benzyloxycarbonyl-2',2'-dimethylpropionamidophenyl)]porphyrin was obtained in a 59% yielkd.

Field desorption mass spectrum: $(M+H)^{+\cdot}$ = 1547 (molecular weight for $C_{96}H_{90}N_8O_{12}$: 1546).

Infrared absorption spectrum (KBr disc): 3,430, 1,730, 1,680, 1,580, 1,513, 1,450, 1,350, 1,190, 1,112, 970, 800, 760, 740, 700 $cm^{-1}$.

Visible absorption spectrum ($CHCl_3$): $\lambda_{max}$ = 417, 512, 543, 584, 642 nm.

$^1H$—NMR spectrum ($CDCl_3$): δ (ppm) = −2.63 (2H, s), −0.11 (24H, s), 2.09 (8H, s), 4.72 (8H, s), 7.1—8.7 (40H, m), 8.74 (8H, s).

$^{13}C$—NMR spectrum ($CDCl_3$):

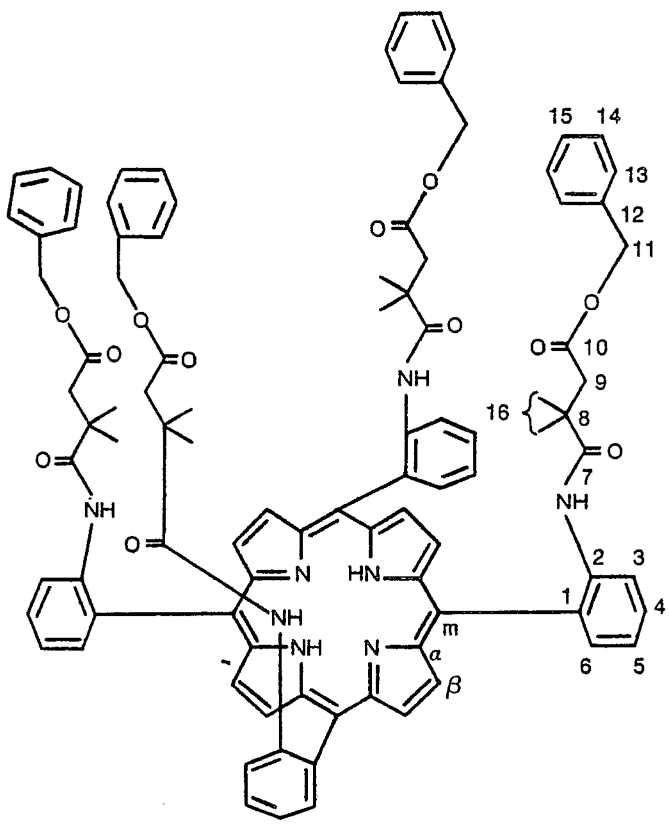

| Position | δ (ppm) | Position | δ (ppm) |
|---|---|---|---|
| α | 147.16 | 7 | 174.72 |
| β | 131.69 | 8 | 43.33 |
| m | 114.89 | 9 | 40.86 |
| 1 | 131.69 | 10 | 170.43 |
| 2 | 138.26 | 11 | 65.93 |
| 3 | 121.65 | 12 | 135.56 |
| 4 | 129.81 | 13 | 127.75 |
| 5 | 123.29 | 14 | 128.40 |
| 6 | 134.56 | 15 | 128.05 |
| | | 16 | 24.42 |

Elemental analysis (wt.%): C, 74.69 (74.49); H, 5.91 (5.86); N, 7.28 (7.24). [The values given in parentheses represent the calculated values for $C_{96}H_{90}N_8O_{12}$.]

Employing the procedure of Example 24, iron was introduced by reacting 0.3 g of 5,10,15,20-tetra[α,α,α,α-o-(3′-benzyloxycarbonyl-2′,2′-dimethylpropionamido)phenyl]porphyrin, 0.2 ml of pyridine, and 0.5 g of ferrous bromide dihydrate ($FeBr_2.2H_2O$). Subsequently, the reaction mixture was worked up in the same manner as in Example 24. The resulting product was recrystallized from a mixture of dichloromethane, ether and petroleum ether. Thus, 0.26 g of bromo[5,10,15,20-tetra{α,α,α,α-o-(3′-benzyloxycarbonyl-2′,2′-dimethylpropanamido)phenyl}porphyrinato]iron(III) (hereinafter referred to as Fe(TproPP.$COOCH_2Ph$)Br) was obtained in an 81% yield.

Field desorption mass spectrum: $(M+H)^+$ = 1681 (molecular weight for $C_{96}H_{88}N_8O_{12}FeBr$: 1680).

Infrared absorption spectrum (KBr disc): 3,430, 2,960, 1,740, 1,690, 1,585, 1,520, 1,446, 1,170, 1,000, 805, 760, 700 $cm^{-1}$.

Visible absorption spectrum ($CHCl_3$): $\lambda_{max}$ = 413, 506, 577, 650 nm.

Elemental analysis (wt.%): C, 67.34 (67.23); H, 5.11 (5.20); N, 6.28 (6.50). [The values given in parentheses represent the calculated values for $C_{96}H_{88}N_8O_{12}FeBr.1/2CH_2Cl_2$.]

## Example 27

The procedure of Example 13 was repeated except that the Fe(TproPP.$COOCH_2Ph$)Br synthesized in Example 26 was used as an iron(III) porphyrin. Thus, there was obtained an aqueous dispersion of liposomes having the iron(II)-5,10,15,20-tetra[α,α,α,α-o-(3′-benzyloxycarbonyl-2′,2′-dimethylpropanamido)phenyl]porphyrin-bis(1-laurylimidazole) complex enclosed therein. The visible absorption spectrum of this dispersion exhibited absorption maxima at 429 and 533 nm. As soon as oxygen gas was bubbled through the dispersion, there occurred a shift to a spectrum ($\lambda_{max}$ = 425 and 537 nm) corresponding to the oxygenated complex. Furthermore, the original spectrum ($\lambda_{max}$ = 429 and 533 nm) was reversibly obtained by bubbling nitrogen gas through the same dispersion. The half-life of the oxygenated complex was found to be 1 hour.

Example 28

The procedure of Example 12 was repeated except that 2,2-dimethylhexadecanoyl chloride was used in place of the 2,2-dimethyltetradecanoyl chloride. Thus, 5,10,15,20-tetra[α,α,α,α-o-(2',2'-dimethylhexadecanamido)phenyl]porphyrin was obtained in a 24% yield.

Visible absorption spectrum ($CCl_4$): $\lambda_{max}$ = 417, 510, 542, 586, 652 nm.

Infrared absorption spectrum ($CCl_4$ solution): 3,450, 3,330, 2,960, 2,925, 2,860, 1,700, 1,580, 1,515 $cm^{-1}$.

Field desorption mass spectrum: $M^{+\cdot}$ = 1740 (molecular weight for $C_{116}H_{170}N_8O_4$: 1738).

$^{13}C$—NMR spectrum ($CDCl_3$, TMS):

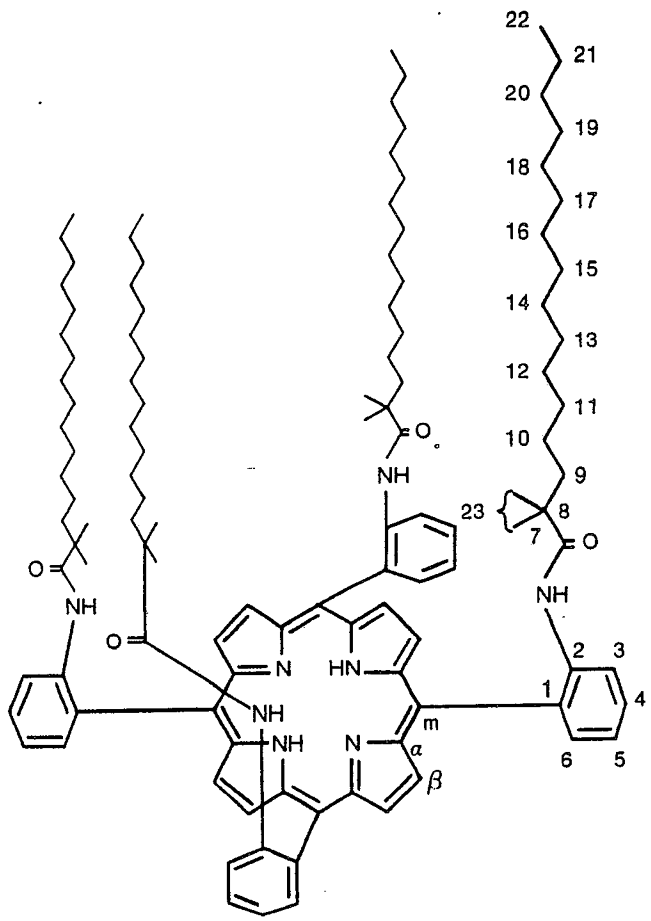

| Position | δ (ppm). | Position | δ (ppm) |
|---|---|---|---|
| α | 147.13 | 7 | 174.84 |
| β | 131.87 | 8 | 42.36 |
| m | 114.87 | 9 | 41.10 |
| 1 | 130.63 | 10 | 24.95 |
| 2 | 138.47 | 11—19 | 29.41<br>29.71<br>30.03 |
| 3 | 120.85 | | |
| 4 | 130.07 | 20 | 31.94 |
| 5 | 123.02 | 21 | 22.72 |
| 6 | 134.24 | 22 | 14.15 |
| | | 23 | 24.16 |

Elemental analysis (wt.%): C, 80.01 (80.04); H, 10.08 (9.84); N, 6.33 (6.44). [The values given in parentheses represent the calculated values for $C_{116}H_{170}N_8O_4$.]

Employing the procedure of Example 12, iron was introduced into the center of the above prophyrin. Thus, bromo[5,10,15,20-tetra{α,α,α,α-o-(2',2'-dimethylhexadecanamido)phenyl}porphyrinato]-iron(III) was obtained in a 67% yield.

Visible absorption spectrum ($CCl_4$): $\lambda_{max}$ = 415, 508, 583 nm.

Field desorption mass spectrum: $M^+$ = 1874 (molecular weight for $C_{116}H_{168}N_8O_4FeBr$: 1872 on the assumption that Fe = 57 and Br = 79).

Infrared absorption spectrum ($CCl_4$): 3,440, 2,930, 2,860, 1,695, 1,585, 1,512, 1,440, 1,300, 1,000, 805, 755, 722 $cm^{-1}$.

Elemental analysis (wt.%): C, 74.21 (74.33); H, 9.28 (9.03); N, 5.89 (5.98). [The values given in parentheses represent the calculated values for $C_{116}H_{168}N_8O_4FeBr$.]

Example 29

The procedure of Example 13 was repeated except that the bromo[5,10,15,20-tetra{α,α,α,α-o-(2',2'-dimethylhexadecanamido)-phenyl}porphynato]iron(III) synthesized in Example 28 was used as an iron(III) porphyrin. Thus, there was obtained an aqueous dispersion of liposomes having the iron(II)-5,10,15,20-tetra[α,α,α,α-o-(2',2'-dimethylhexadecanamido)phenyl]porphyrin-bis(1-laurylimidazole) complex enclosed

therein. The formation of the oxygenated complex was confirmed in the same manner as in Example 13. The visible absorption spectrum of the deoxygenated form of this complex exhibited an absorption maximum at 533 nm, while that of the oxygenated complex had a $\lambda_{max}$ value of 536 nm. The half-life of the oxygenated complex was found to be more than 4 hours.

Example 30

The procedure of Example 12 was repeated except that 2,2-dimethyl butanoyl chloride was used as an acid chloride. Thus, there was obtained 5,10,15,20-tetra[α,α,α,α-o-(2′,2′-dimethylbutanamidophenyl)]porphyrin in 53% yield.

Visible absorption spectrum ($CHCl_3$) 416, 510, 542, 585, 648 nm.

Infrared absorption spectrum (KBr disc): 3,450, 3,330, 2,980, 1,690, 1,587, 1,450, 1,300, 1,160, 975, 805, 760 $cm^{-1}$.

$^1H$—NMR spectrum ($CDCl_3$): δ (ppm) = −2.60 (2H, s), −0.05 (12H, t; J = 7Hz), 0.02 (24H, s), 0.61 (8H, q; J = 7Hz), 7.26—8.00 (16H, m), 8.83 (8H, s).

$^{13}C$—NMR spectrum ($CDCl_3$, TMS):

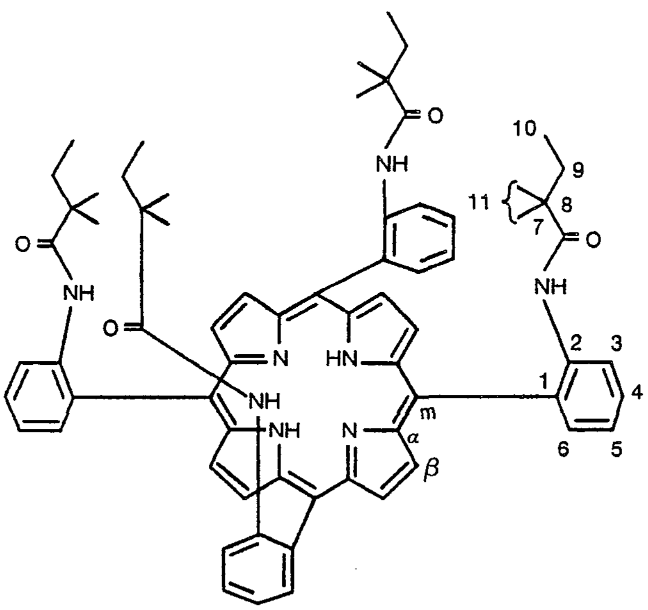

| Position | δ (ppm) | Position | δ (ppm) |
|---|---|---|---|
| α | 147.00 | 7 | 174.86 |
| β | 131.66 | 8 | 42.68 |
| m | 114.98 | 9 | 33.14 |
| 1 | 130.89 | 10 | 8.45 |
| 2 | 138.26 | 11 | 23.87 |
| 3 | 120.91 | | |
| 4 | 129.99 | | |
| 5 | 122.99 | | |
| 6 | 134.41 | | |

Field desorption Mass spectrum: (M+1) 1067 ($C_{68}H_{72}N_8O_4$ = 1066).

The procedure of Example 12 for iron-insertation into a porphyrin was repeated except that the 5,10,15,20-tetra[α,α,α,α-o-(2′,2′-dimethylbutanamidophenyl)]porphyrin (0.19 gr.) was used as a porphyrin. Thus, there was obtained bromo{5,10,15,20-tetra[α,α,α,α-o-(2′,2′-dimethylbutanamidophenyl)]porphinato} iron(III). The yield was 0.13 gr (61%).

Visible absorption spectrum ($CHCl_3$) 416, 508, 586, 654 nm.

Infrared absorption spectrum (KBr disc): 3,440, 2,975, 1,690, 1,585, 1,510, 1,443, 1,295, 1,150, 1,000, 805, 760 $cm^{-1}$.

Field desorption mass spectrum: (M+1) 1201 ($C_{68}H_{72}N_8O_4FeBr$ = 1200).

Elemental analysis (wt%): C 68.19 (67.99), H 6.25 (6.04), N 9.02 (9.33). (The values in the parentheses were the calculated ones for $C_{68}H_{72}N_8O_4FeBr$.)

Example 31

The procedure of Example 1 for the preparation of a liposomal aqueous solution was repeated except that the bromo{5,10,15,20-tetra-[α,α,α,α-o-(2′,2′-dimethylbutanamidophenyl)]porphinato} iron(III) was used as an iron(III)porphyrin. Thus, there was obtained an aqueous dispersion of liposomes having the iron(II)-5,10,15,20-tetra[α,α,α,α-o-(2′,2′-dimethylbutanamidophenyl)]porphyrin-mono(1-n-lauryl-2-methylimidazole) complex enclosed therein. The visible absorption spectrum of this dispersion recorded under an atmosphere of nitrogen gas exhibited absorption maxima at 533 and 562 nm and corresponded to the deoxygenated form of the complex. The spectrum due to the oxygenated complex was obtained immediately after bubbling oxygen gas through the deoxy solution. The spectrum exhibited an absorption maximum at 543 nm. By bubbling nitrogen gas through the oxygenated solution, a shift to the deoxygenated spectrum was observed reversibly. The life-time of the oxygenated complex at room temperature was found to be more than about 3 hours.

Example 32

The procedure of Example 12 for the preparation of 2,2-dimethyl-alkanoyl chloride was repeated except that the stearyl bromide was used as an alkylbromide. Then, the thus obtained 2,2-dimethyl eiconsanoic acid chloride (2.0 g) was reacted with α,α,α-TamPPH$_2$ (0.21 g) in a manner similar to that in Example 12. Thus, there was obtained 5,10,15,20-tetra[α,α,α,α-o-(2′,2′-dimethyleicosanamidophenyl)]-porphyrin. The yield was 37%.

Visible absorption spectrum (CHCl$_3$): 418, 510, 545, 587, 651 nm.

Infrared absorption spectrum (KBr disc): 3,440, 2,925, 2,860, 1,690, 1,583, 1,510, 1,470, 1,450, 1,300, 1,150, 970, 805, 760, 725 cm$^{-1}$.

$^1$H—NMR spectrum (CDCl$_3$, TMS): δ (ppm) = −2.59 (2H, s), −0.21 (24H, s), 1.26 (128H, s), 7.13—7.94 (16H, m), 8.72 (4H, s), 8.82 (8H, s).

$^{13}$C—NMR spectrum (CDCl$_3$, TMS):

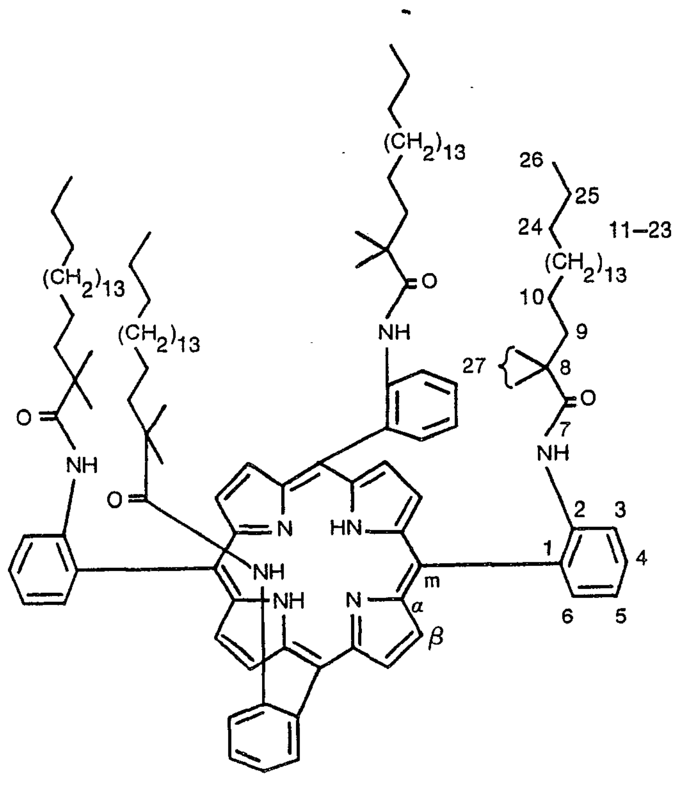

| Position | δ (ppm) | Position | δ (ppm) |
|---|---|---|---|
| α | 147.30 | 7 | 174.78 |
| β | 131.75 | 8 | 42.36 |
| m | 114.87 | 9 | 41.07 |
| 1 | 130.57 | 11—23 | 29.36, 29.65, 30.03 |
| 2 | 138.50 | 24 | 31.91 |
| 3 | 120.83 | 25 | 22.69 |
| 4 | 130.04 | 26 | 14.09 |
| 5 | 122.97 | 27 | 24.16 |
| 6 | 134.24 | | |

The procedure of Example 12 for iron-insertion into a porphyrin was repeated except that the 5,10,15,20-tetra[α,α,α,α-o-(2′,2′-dimethyleicosanamidophenyl)]porphyrin was used as a porphyrin. Thus, there was obtained bromo{5,10,15,20-tetra[α,α,α,α-o-(2′,2′-dimethyleicosanamidophenyl)]porphinato}-iron(III]. The yield was 73%.

Visible absorption spectrum ($CHCl_3$): 416, 508, 586, 652, 684 nm.

Infrared absorption spectrum (KBr disc): 3,440, 2,940, 2,860, 1,695, 1,585, 1,513, 1,460, 1,440, 1,300, 1,145, 1,000, 805, 765, 730 $cm^{-1}$.

Elemental analysis (wt%): C 75.58 (75.54), H 9.91 (9.61), N 5.19 (5.34). (The values in the parentheses were the calculated ones for $C_{132}H_{200}N_8O_4FeBr$.)

Example 33

The procedure of Example 13 for the preparation of a liposomal aqueous solution was repeated except that the bromo{5,10,15,20-tetra-[α,α,α,α-o-(2',2'-dimethyleicosanamidophenyl)]porphinato}iron(III) was used as an iron(III)porphyrin. Thus, there was obtained the aqueous dispersion of liposomes having the iron(II)-5,10,15,20-tetra[α,α,α,α-o-(2',2'-dimethyleicosanamidophenyl)]porphyrin-bis(1-n-laurylimidazole) complex enclosed therein. The visible absorption spectrum of this dispersion recorded under an atmosphere of nitrogen gas exhibited an absorption maximum at 532 nm and corresponded the deoxygenated form of the complex. The spectrum due to the oxygenated complex was observed immediately after bubbling oxygen gas through the deoxygenated solution. The spectrum exhibited an absorption maximum at 536 nm. The life-time of the oxygenated complex at room temperature was found to be more than 4 hours.

**Claims**

1. An oxygen-adsorbing and desorbing agent consisting of a liposome formed from at least one lipid or a mixture of said lipid and cholesterol, said liposome having enclosed in a hydrophobic region therof an iron(II)-5,10,15,20-tetra(α,α,α,α-o-substituted amidophenyl)porphyrin-imidazole complex having the formula

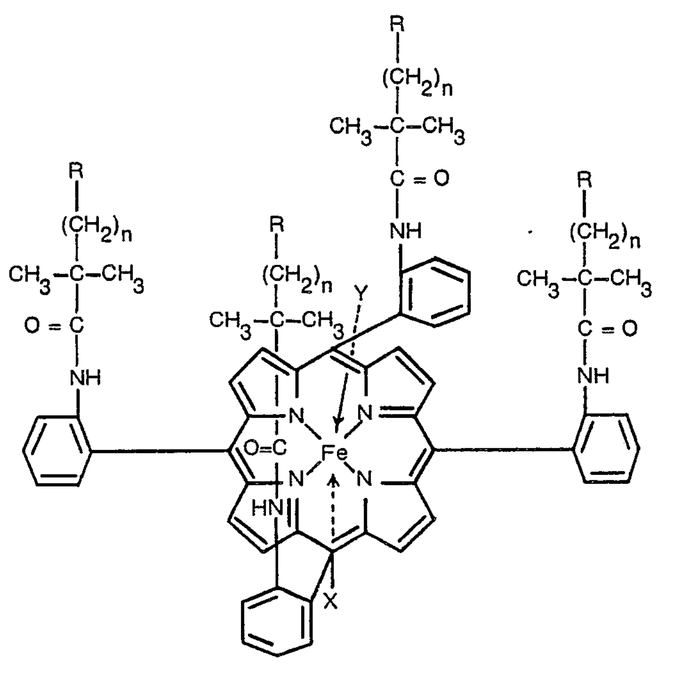

(I)

wherein R is a hydrogen atom, an alkoxycarbonyl group having 1 to 5 carbon atoms in the alkoxy moiety, a benzyloxycarbonyl group or a carboxyl group, n is an integer of 0 to 20, wherein when R is hydrogen, n is not 0, and each of X and Y is a substituted imidazole having the formula:

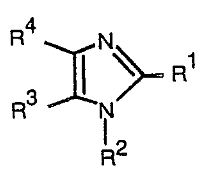

wherein $R^1$ is a hydrogen atom or an alkyl group having one to three carbon atoms, and each of $R^2$, $R^3$ and $R^4$ is independently a hydrogen atom or a hydrophobic group, at least one of $R^2$, $R^3$ and $R^4$ is a hydrophobic group, and wherein when $R^1$ is an alkyl group, Y is not present, said liposome being dispersed in an aqueous medium.

2. The agent of claim 1, wherein R is represented by the formula —COOR', wherein R' is a hydrogen atom, an alkyl group having 1 to 5 carbon atoms or a benzyl group.

3. The agent of claim 1, wherein the hydrophobic group is represented by the formula $-(CH_2)_l-Z$ wherein 1 is an integer of 10 to 20 and Z is a hydrogen atom or an alkoxycarbonyl group having 1 to 5 carbon atoms in the alkoxy moiety.

4. The agent of claim 1, wherein the hydrophobic group is a trityl group.

5. The agent of claim 1, wherein $R^2$ is a hydrophobic group.

6. The agent of any one of claims 1 to 5, wherein $R^1$ is hydrogen and the molar ratio of the substituted imidazole to the iron-porphyrin is from 2 to 30.

7. The agent of any one of claims 1 to 5, wherein $R^1$ is an alkyl group, and the molar ratio of the imidazole to the iron-porphyrin is from 10 to 100.

8. The agent of any one of claims 1 to 5, wherein the molar ratio of said lipid or said mixture containing at least one lipid and cholesterol to the iron-porphyrin is from 10 to 1,000.

9. The agent of claim 1, wherein said lipid is a phospholipid or a sphingolipid.

10. The agent of claim 1, wherein the weight ratio of cholesterol to the lipid is 1:2 to 1:20.

**Patentansprüche**

1. Sauerstoffadsorptions- und -desorptionsmittel, bestehend aus einem Liposom, welches aus mindestens einem Lipid oder einem Gemisch des genannten Lipids und Cholesterin gebildet wird, wobei das Liposom in einem hydrophoben Bereich desselben einen Eisen(II)-5,10,15,20-tetra-(α,α,α,α-o-substituierten Amidophenyl)porphyrin-imidazol-Komplex der folgenden Formel eingeschlossen enthält:

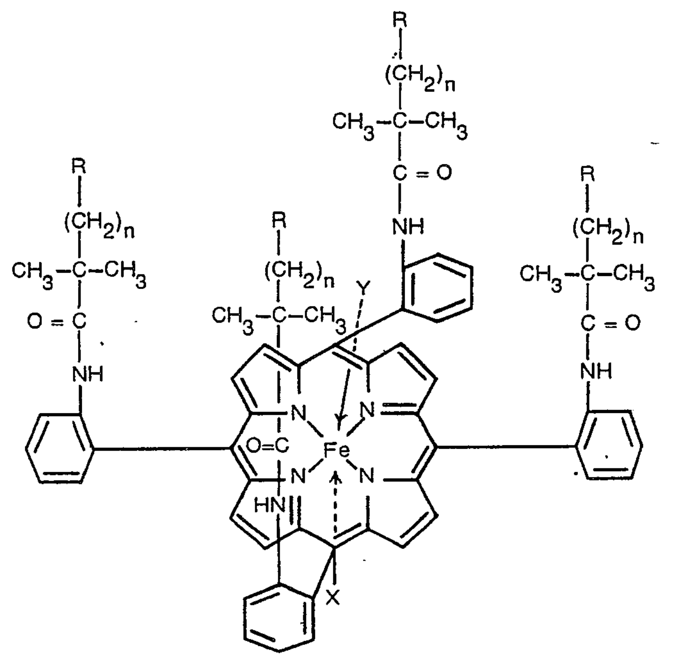

(I)

worin R ein Wasserstoffatom, eine Alkoxycarbonylgruppe mit 1 bis 5 Kohlenstoffatomen in der Alkoxykomponente, eine Benzyloxycarbonylgruppe oder eine Carboxylgruppe darstellt, n eine ganze Zahl von 0 bis 20 bedeutet, wobei, wenn R Wasserstoff ist, n nicht 0 bedeutet, und jeder der Substituenten X und Y ein substituiertes Imidazol der folgenden Formel darstellt:

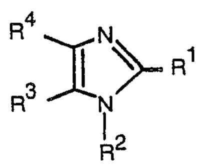

worin $R^1$ ein Wasserstoffatom oder eine Alkylgruppe mit 1 bis 3 Kohlenstoffatomen bedeutet, und jeder der Substituenten $R^2$, $R^3$ und $R^4$ unabhängig voneinander ein Wasserstoffatom oder eine hydrophobe Gruppe bedeutet, wobei mindestens einer der Substituenten $R^2$, $R^3$ und $R^4$ eine hydrophobe Gruppe darstellt, vorausgesetzt, dass wenn $R^1$ eine Alkylgruppe bedeutet, Y nicht anwesend ist, wobei das genannte Liposom in einem wässrigen Medium dispergiert ist.

2. Mittel nach Anspruch 1, worin R die Formel —COOR′ darstellt, wobei R′ ein Wasserstoffatom, eine Alkylgruppe mit 1 bis 5 Kohlenstoffatomen oder eine Benzylgruppe bedeutet.

3. Mittel nach Anspruch 1, worin die hydrophobe Gruppe die Formel $-(CH_2)_l-Z$ bedeutet, wobei 1 eine ganze Zahl von 10 bis 20 bedeutet und Z ein Wasserstoffatom oder eine Alkoxycarbonylgruppe mit 1 bis 5 Kohlenstoffatomen in der Alkoxykomponente darstellt.

4. Mittel nach Anspruch 1, worin die hydrophobe Gruppe eine Tritylgruppe darstellt.

5. Mittel nach Anspruch 1, worin $R^2$ eine hydrophobe Gruppe darstellt.

6. Mittel nach einem oder mehreren der Ansprüche 1 bis 5, worin $R^1$ Wasserstoff bedeutet und das Molverhältnis des substituierten Imidazol zum Eisen-porphyrin von 2 bis 30 beträgt.

7. Mittel nach einem oder mehreren der Ansprüche 1 bis 5, worin $R^1$ eine Alkylgruppe bedeutet, und das Molverhältnis von Imidazol zu Eisen-porphyrin von 10 bis 100 beträgt.

8. Mittel nach einem oder mehreren der Ansprüche 1 bis 5, worin das Molverhältnis des genannten Lipids oder des genannten Gemisches, welches mindestens ein Lipid und Cholesterin enthält, zu Eisen-porphyrin von 10 bis 1.000 beträgt.

9. Mittel nach Anspruch 1, worin das genannte Lipid ein Phospholipid oder ein Sphingolipid darstellt.

10. Mittel nach Anspruch 1, worin das Gewichtsverhältnis von Cholesterin zu Lipid 1:2 bis 1:20 beträgt.

**Revendications**

1. Agent d'adsorption et de désorbtion de l'oxygène consistant en un liposome formé à patir d'au moins un lipide ou un mélange dudit lipide et de cholestérol, ledit liposome renfermant dans une region hydrophobe un complexe fer (II)-5,10,15,20-tétra-(α,α,α,α-o-amido substitué-phényle)porphyrine-imidazole répondant à la formule:

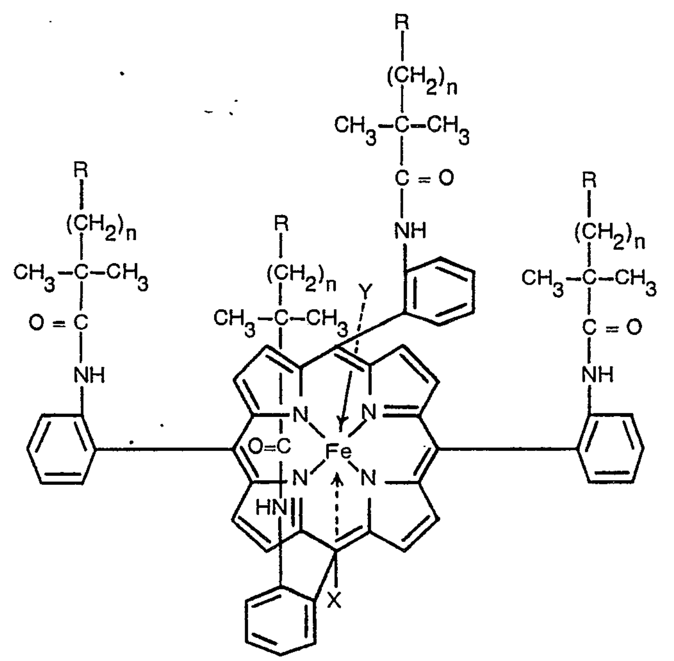

(I)

dans laquelle R est un atome d'hydrogène, un groupe (alcoxy en $C_1$—$C_5$)-carbonyle, un groupe benzoxycarbonyle ou un groupe carboxyle, n est un entier de 0 à 20, dans laquelle n n'est pas égal à 0, lorsque R est l'hydrogène, et X et Y représentent chacun un imidazole substitué de formule:

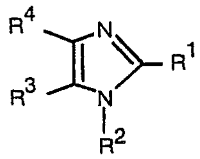

dans laquelle $R^1$ est un atome d'hydrogène ou un groupe alkyle en $C_1$—$C_3$ et $R^2$, $R^3$ et $R^4$ représentent chacun indépendamment un atome d'hydrogène ou un groupe hdyrophobe, l'un au moins des restes $R^2$, $R^3$ et $R^4$ est un groupe hydrophobe, et dans laquelle, lorsque $R^1$ est un groupe alkyle, Y n'est pas présent, ledit liposome étant dispersé dans un milieu aqueux.

2. Agent selon la revendication 1, dans lequel R est représenté par la formule —COOR', dans laquelle R' est un atome d'hydrogène, un groupe alkyle en $C_1$—$C_5$ ou un groupe benzyle.

3. Agent selon la revendication 1, dans lequel le groupe hydrophobe est représenté par la formule $-(CH_2)_l-Z$ dans laquelle 1 est un entier de 10 à 20 et Z est un atome d'hydrogène ou un groupe (alcoxy en $C_1-C_5$) carbonyle.

4. Agent selon la revendication 1, dans lequel le groupe hydrophobe est un groupe trityle.

5. Agent selon la revendication 1, dans lequel $R^2$ est un groupe hydrophobe.

6. Agent selon l'une quelconque des revendications 1 à 5, dans lequel $R^1$ est l'hydrogène et le rapport molaire de l'imidazole substitué au fer-porphyrine est de 2 à 30.

7. Agent selon l'une quelconque des revendications 1 à 5, dans lequel $R^1$ est un groupe alkyle et le rapport molaire de l'imidazole au fer-porphyrine est de 10 à 100.

8. Agent selon l'une quelconque des revendications 1 à 5, dans lequel le rapport molaire dudit lipide ou dudit mélange contenant au moins un lipide et du cholestérol au fer-porphyrine est de 10 à 1000.

9. Agent selon la revendication 1, dans lequel ledit lipide est un phospholipide ou un sphingolipide.

10. Agent selon la revendication 1, dans lequel le rapport pondéral du cholestérol au lipide est de 1:2 à 1:20.

F I G. 1

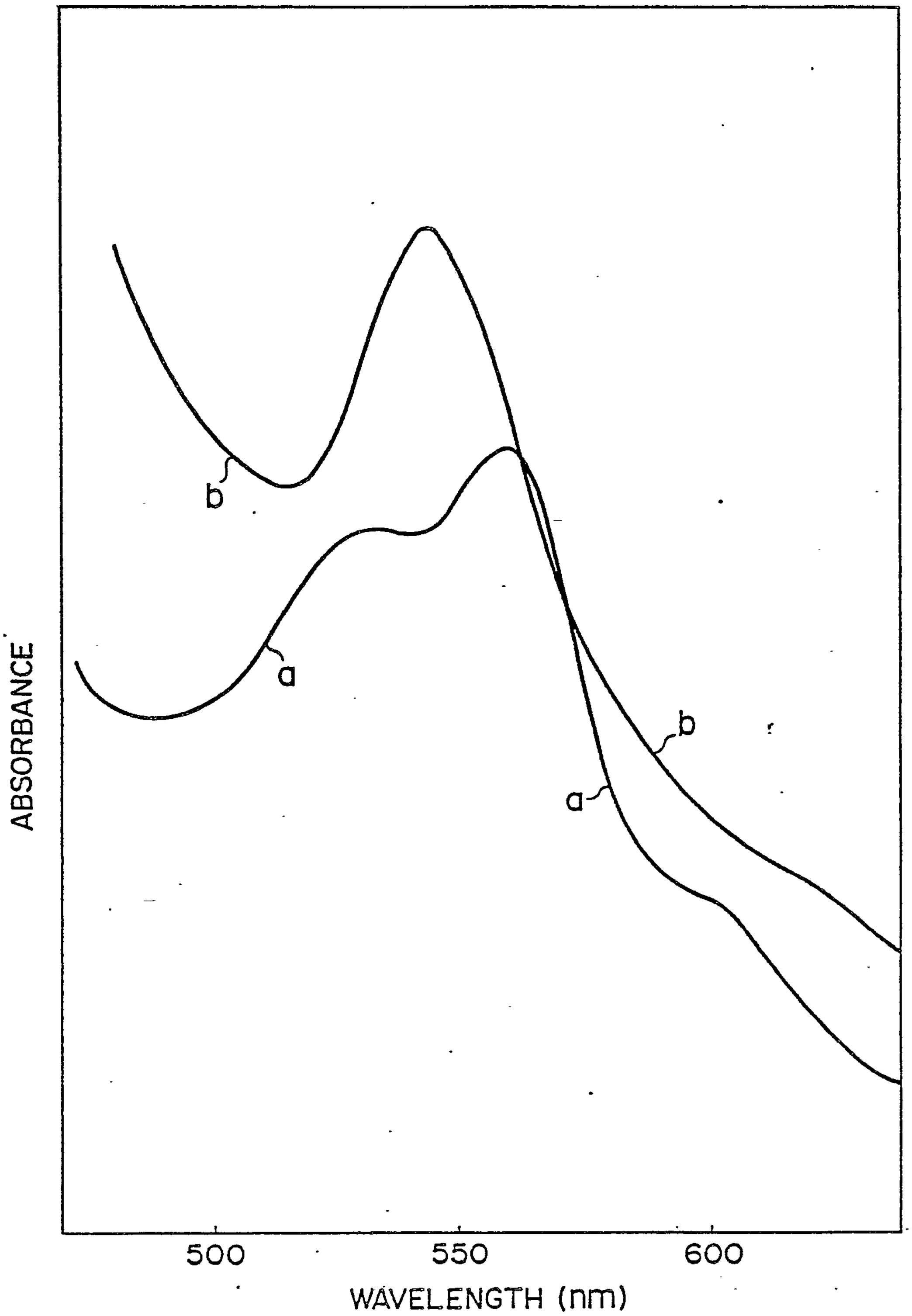

F I G. 2